(19) **Europäisches Patentamt / European Patent Office / Office européen des brevets**

(11) **EP 3 345 494 A1**

(12) **EUROPEAN PATENT APPLICATION**
published in accordance with Art. 153(4) EPC

(43) Date of publication:
**11.07.2018 Bulletin 2018/28**

(51) Int Cl.:
*A23L 29/20* (2016.01)    *A23G 9/04* (2006.01)
*A23G 9/32* (2006.01)    *A23G 9/44* (2006.01)
*A23G 9/52* (2006.01)    *A23L 15/00* (2016.01)

(21) Application number: **16842037.0**

(22) Date of filing: **02.09.2016**

(86) International application number:
**PCT/JP2016/075915**

(87) International publication number:
**WO 2017/039008 (09.03.2017 Gazette 2017/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **02.09.2015   JP 2015172486**
**05.10.2015   JP 2015197303**
**09.10.2015   JP 2015201488**
**06.04.2016   JP 2016076613**

(71) Applicant: **San-Ei Gen F.F.I., INC.**
**Toyonaka-shi, Osaka 561-8588 (JP)**

(72) Inventors:
• **NAKAJIMA, Kohei**
**Toyonaka-shi**
**Osaka 561-8588 (JP)**
• **GOTO, Kayo**
**Toyonaka-shi**
**Osaka 561-8588 (JP)**
• **OKUDA, Eiji**
**Toyonaka-shi**
**Osaka 561-8588 (JP)**

• **SAITO, Ariko**
**Toyonaka-shi**
**Osaka 561-8588 (JP)**
• **KURODA, Hiroaki**
**Toyonaka-shi**
**Osaka 561-8588 (JP)**
• **FUJITA, Yasuyuki**
**Toyonaka-shi**
**Osaka 561-8588 (JP)**
• **ONODERA, Makoto**
**Toyonaka-shi**
**Osaka 561-8588 (JP)**
• **SATO, Hiroyuki**
**Toyonaka-shi**
**Osaka 561-8588 (JP)**
• **NAKAO, Satomi**
**Toyonaka-shi**
**Osaka 561-8588 (JP)**
• **KOMIYA, Yugo**
**Toyonaka-shi**
**Osaka 561-8588 (JP)**
• **TAKESONO, Minami**
**Toyonaka-shi**
**Osaka 561-8588 (JP)**

(74) Representative: **Hoffmann Eitle**
**Patent- und Rechtsanwälte PartmbB**
**Arabellastraße 30**
**81925 München (DE)**

(54) **METHOD FOR IMPROVING OR MAINTAINING PHYSICAL PROPERTIES OF SUBSTANCE**

(57)    An object of the present invention is to provide a means for improving or maintaining physical properties of a substance (in particular, a composition) (e.g., food). The object is achieved by a foam-containing composition that contains welan gum.

Printed by Jouve, 75001 PARIS (FR)

EP 3 345 494 A1

**Description**

Technical Field

The present invention relates to a method for improving or maintaining physical properties of a substance (in particular, a composition) (e.g., food). More specifically, the present invention relates to the following technical fields 1 to 3.

1. The present invention relates to a foam-containing composition, in particular, a foam-containing composition with improved foam stability.
2. The present invention relates to a method for improving the texture of an egg white-containing baked confection.
3. The present invention relates to a frozen dessert, and a method for stabilizing the frozen dessert.

Background Art

The following is the background art of the technical fields 1 to 3.

Regarding the technical field 1, an example of the foam-containing composition is a foam-containing food. The foam-containing food includes whipped cream, whipped desserts, foamed beverages, and beverages containing layered foam. As noted here, a variety of foods are combined with foam. Whipped cream, which is a foam-containing food, is widely used in toppings for desserts or western confectionery, such as cake, pudding, and mousse.

Whipped cream includes the following:

(1) Whipped cream made from cream of milk origin, such as cow milk (e.g., fresh cream); and
(2) "Artificial cream" (non-milk cream) made by mixing fat other than milk fat with skimmed milk, skimmed milk powder, an emulsifier, and/or a flavor, etc.

Whipped desserts broadly refer to food containing foam, and examples thereof include mousse, *awayukikan* (fluffy snow-like Japanese jelly sweets), bavarois, and marshmallow. These may be an individual single food (e.g., a mousse dessert) or may be combined with jelly, pudding, or the like.

In the field of beverages as well, beverages with smooth mouthfeel have been provided by the following means:

(1) adding foam produced by whipping the above-mentioned cream or milk components, and the like; or
(2) forming foam of carbon dioxide by mixing a powdery mixture containing a foaming component with a liquid, such as water or fruit juice.

In order to impart the shape retention, prevent syneresis, or improve the bodying sensation of whipped cream, polysaccharide thickeners have conventionally been used as a stabilizer. For instance, the use of hydroxypropylcellulose as a stabilizer is known, and the use of hydroxypropylcellulose further in combination with an emulsifier, such as monoglycerin fatty acid ester, diglycerin fatty acid ester, or sorbitan fatty acid ester, is known (e.g., Patent Literature 1-1 and Patent Literature 1-2). However, these techniques have drawbacks, such as (1) difficulty in foaming (overrun), (2) decreased shape retention of foam, (3) increased syneresis, along with reduced edge sharpness of cream when squeezed from a container with a flower-shaped (or star-shaped) nozzle, deletion of gloss, and the like.

Another example of proposed techniques is a low-fat cream that contains a high-molecular substance selected from microcrystalline cellulose and pullulan, with the overrun after whipping being 200% or less and the fat content being 40 wt% or less (Patent Literature 1-3). However, this technique has drawbacks, such as (1) the need for a long whipping time and (2) stability issues, such as imparting fluffy and non-resilient structure over time.

When whipped cream is prepared, sterilization may be performed before whipping, and the sterilization performed is UHT sterilization (Patent Literature 1-4). However, when UHT treatment is performed, the following problems arise: it takes time to achieve a sufficient overrun; the viscosity of the mix increases; the shape retention after whipping is undermined; and syneresis becomes more likely to occur.

Because of these problems, there has been demands for compositions that can impart properties required for whipped cream, such as excellent foamability with high overrun even after UHT sterilization, shape retention, a syneresis-prevention effect, and improvement of bodying sensation.

Whipped desserts have a lower fat and oil content than that of whipped cream. Thus, a foaming agent and/or a stabilizer is used in whipped desserts.

Gelatin is a foaming agent that has both foamability and foam stability, and is also a gelling agent that exhibits excellent self-restorability of gel. Gelatin is widely used in producing foaming foods, such as whipped desserts. In a typical method for producing a gelatin-containing whipped dessert, a gelatin-containing solution that has once been heated for dissolution is cooled down to about 10°C to form a gel, and crushed with stirring for foaming inside the whipped dessert. At this stage, the gel is reconstituted, with the foam contained inside the gel, thereby giving an excellent whipped dessert.

However, when a gelling agent is used in the production of foam-containing foods, such as whipped desserts, whipping must be performed at or below the melting temperature of the gel. The use of gelatin, for example, has the following drawbacks: stirring at 40°C or below (preferably 10°C or below) is necessary to form foam inside a food; additionally, when allowed to stand at room temperature, the produced foam-containing food melts, and thereby the gas is released from inside.

Prior art discloses a foaming food containing gelatin, native gellan gum, and deacylated gellan gum (Patent Literature 1-5). This technique enables whipping at a high temperature of 45 to 65°C.

For foam-containing beverages, such as cappuccino and milkshakes, various methods have been studied to make the texture (mouthfeel) smooth and impart an excellent texture by forming dense foam in the upper layer or inside of coffee or milk beverages.

Examples of conventional methods for forming foam (foaming methods) include:

(i) a method for forming foam by adding an emulsifier and ethyl alcohol to a beverage, and forcefully mixing them with gas (Patent Literature 1-6);

(ii) a method using, (a) an emulsifier comprising either sorbitan monosaturated fatty acid ester or propylene glycol fatty acid ester, or both, and (b) an emulsifier comprising at least one of glycerin dibasic acid fatty acid ester, citric acid monoglycerine ester, polyglycerin fatty acid ester, or sucrose fatty acid ester (Patent Literature 1-7);

(iii) a method including adding, to a coffee extract, a dairy component in an amount that gives a milk fat content of 0.05 wt% or more of the total amount of the resulting coffee beverage, together with a foaming agent (Patent Literature 1-8);

a method including adding, to a liquid ingredient in the production step of a milk-containing beverage,

(iv) a dairy component in an amount that gives a milk fat content of 0.1 wt% or less of the total amount of the resulting beverage together with a foaming agent (Patent Literature 1-9);

a method including adding a milk peptide and water-soluble hemicellulose to a beverage, filling the resulting beverage into a container, and then shaking it (Patent Literature 1-10);

(v) an instant beverage having a cavity filled with a foaming agent, which forms foam when in contact with water (Patent Literature 1-11);

an effervescent powdered drink mix with excellent foamability that contains carbonates and an organic acid as effervescent components, that is granulated with a fat being solid at room temperature, and whose softening point is between 24°C to 40°C (Patent Literature 1-12); and

(vi) a composition containing a) a foaming component that releases gas upon reconstitution and b) a powdered drink or food or its components having a retarded solubility (Patent Literature 1-13).

However, any of these methods is unsatisfactory in forming fine and dense foam inside of as well as in the upper layer of a beverage liquid, and in stably maintaining the formed foam inside the beverage liquid.

Specifically, these methods have the following drawbacks:

(1) the foam formed inside the beverage liquid immediately rise to the surface of the beverage, and are unlikely to be retained inside the beverage liquid; and

(2) (a) when the beverage is poured from a container, such as a bottle, to a container, such as a cup, and (b) when the beverage in a container is drunk, only the liquid part of the beverage flows out, and the foam, which is of importance, remain in the container, and beverage consumers are unable to enjoy the smooth texture.

Another example of the foam-containing composition is a washing agent. As typically understood, a washing agent forms foam, and can be used for a washing purpose. A solvent suitable for laundry and various components are mixed in a washing agent, and the form of washing agents is designed in view of the convenience in use. In particular, most recent washing agents, including soap, laundry detergents, fabric softeners, and kitchen detergents, are likely to be in the form of liquid products.

A chelating agent, a dispersant, a surfactant, soap, and a polysaccharide, and the like are added to a washing agent. The function of removing stains from an object, which is the main purpose of using washing agents, is largely due to the surfactant. A surfactant adsorbs onto stains, and separates the stains from the object. Thereby the object is washed. During washing, bubbles are formed due to a component contained in the washing agent. Bubbles have the following effects:

(1) prevent the separated stains from readhering to the object; and
(2) prolong the contact time of the surfactant and the like with stain components.

As a method for improving the foamability of a washing agent, a method using a glyceroglycolipid surfactant is disclosed (Patent Literature 1-14). However, heavy use of surfactant affects the environment, and alternative techniques are desired.

Foam of a washing agent is required not to flow down, and to remain in the area where the foam formed (adhesive property of foam).

Methods for improving the adhesiveness of foam include a method for suppressing the drop-off of foam. As a technique related to the method, for example, the following are proposed: a sprayable liquid detergent composition containing a specific cellulose, a surfactant, and water (Patent Literature 1-15); and a sprayable liquid obtained by filling a spraying device with a composition containing specific cellulose microparticles and a liquid dispersant (Patent Literature 1-16).

However, available components in these techniques are limited to specific components; thus, these techniques are somewhat difficult to perform.

Patent Literature 1-17, Patent Literature 1-18, and the like disclose, as another technique, methods for suppressing the drop-off of a washing agent. However, these are all intended to suppress the drop-off of "liquid," and neither enable nor suggest the suppression of drop-off of "foam," unlike the present invention.

Regarding the technical field 2, a variety of food hydrocolloids have been used to improve the quality of food (e.g., physical properties such as the thickening property, gelling property, stability, dispersibility, interfacial activity, or foamability), or to enrich useful components such as dietary fiber (enrichment). Food hydrocolloids refer to particles of proteins, polysaccharides, and the like with a particle size of about 1 μm or less that are present in a food with water as the dispersant. Food hydrocolloids have unique physical properties, and are not only useful as food materials *per se*, but also improve the physical properties and functionality of other foods when added in a small amount to other foods. Texture, which is one of the dominant factor of the palatability of food, is closely related to the physical properties of food. Food hydrocolloids that can control physical properties of food are also called texture modifiers.

Food hydrocolloids have been widely used to improve the hardness or swallowing properties of food for people with chewing difficulties or dysphagia patients, and the needs for these applications are expanding. Additionally, texture-improving agents are receiving increased attention which can not only improve physical properties of foods, but also improve texture of foods, such as imparting softer texture, imparting crispiness, improving swallowing ability, imparting more meaty feeling, improving perceived meltability in mouth, and the like, without greatly varying the palatability when added to food as a texture-improving agent.

To meet diverse needs, there have been demands for food hydrocolloids that have excellent functionality, such as heat resistance, freeze-thaw resistance, acid resistance, and salt tolerance. To respond to the demands, there has been considered a method of using multiple food hydrocolloids that have different functionalities in combination, to employ the resulting complementary or synergistic effect.

For example, effects, such as an increase in gel strength and a decrease in syneresis, that are provided by a combination of xanthan gum and guar gum, a combination of xanthan gum and locust bean gum, a combination of xanthan gum and glucomannan, or the like are known (Non-patent Literature 2-1). However, to meet diverse market needs, novel materials with more sophisticated functionality have been desired.

Welan gum is known for its applicabilities to ink compositions, concrete or cement-based materials, materials for unvulcanized rubber, and cosmetic compositions. Examples of application of welan gum being considered include an ink composition for water-based ballpoint pens (Patent Literature 2-1), cement or concrete compositions containing welan gum (Patent Literature 2-2 and Patent Literature 2-3), an parting agent composition for unvulcanized rubber (Patent Literature 2-4), and a cosmetic composition containing gellan gum or its derivative, a solid compound, and a univalent salt (Patent Literature 2-5).

However, improvement and modification of the physical properties and functionality of food that are achieved by using welan gum therein have not been practically researched.

Regarding the technical field 3, it is typically preferable for frozen desserts to be stored and distributed at -18°C or below at which ice crystals are unlikely to develop and water migration is unlikely to occur. However, during transportation from the manufacturing facility to shops, or when frozen desserts are moved from the warehouse in a shop to the showcase, frozen desserts may be placed at room temperature, and the temperature of the frozen desserts may increase. The temperature inside the freezer in which frozen desserts are stored may also increase by 10°C or more from the set temperature of -18°C or below by opening and closing the freezer. When such a temperature change (heat shock) occurs, a syneresis phenomenon in which moisture generated by melting ice leaks out from the frozen desserts, or a phenomenon in which the sugar constituents leak out from the frozen desserts may occur.

Additionally, a frozen dessert product can be deteriorated by being left at room temperature for a certain period of time, for example, change in shape due to melting or imbalanced concentrations of the ingredients. The frozen dessert product that has melted once does not return to its original state even by refreezing, and its commercial value would be lost.

Thus, frozen desserts are ideally stored and distributed at -18°C or below. However, there may be a case in which the temperature of -18°C or below cannot be kept during the distribution or delivery of frozen dessert products.

Moreover, there has been a demand for frozen desserts resistant to temperature changes caused by opening and closing the freezer, even when the desserts are stored at home.

The problems described above arise not only in the distribution channel of the end products, but also during the production of frozen desserts, because the entire production line cannot be kept at -18°C or below as mentioned above.

Various methods have been studied for improving the quality of frozen desserts, such as solids dispersibility, emulsion stability, foam stability, formability, shape retention, syneresis suppression, and texture, in addition to the heat-shock resistance described above. In particular, many methods for improving physical properties using food polysaccharides have been developed.

Food polysaccharides have unique physical properties. They are not only useful as food ingredients *per se*, but also have functions to improve the physical properties or functionalities of food when added to other food in a small amount. The quality, such as texture and formability, is an essential factor for frozen desserts, and is an important factor that determines palatability. Thus, the physical properties and functionalities of food polysaccharides have profound effects on the production of stable and palatable frozen desserts.

Food polysaccharides have a variety of origins, and their physical properties and functionalities are also various. The origins of food polysaccharides include seeds, roots and stems, tree sap, fruit, seaweed, and microorganisms. The typical substances thereof are as described below:

seed-originating food polysaccharides include galactomannan (e.g., guar gum, tara gum, and locust bean gum), water-soluble hemicellulose, tamarind seed gum, soybean soluble polysaccharides, starch and psyllium seed gum; root and stem-originating food polysaccharides include konjac flour, glucomannan, and starch; tree sap-originating food polysaccharides include gum arabic, gum tragacanth, gum karaya, and gum ghatti; fruit-originating food polysaccharides include pectin (low methoxyl (LM) pectin and high-methoxyl (HM) pectin); seaweed-originating food polysaccharides include agar, carrageenan, and alginates (e.g., alginic acid and alginate salts); microorganism-originating food polysaccharides include xanthan gum, gellan gum, pullulan, succinoglycan, and curdlan; and animal-originating food polysaccharides include gelatin.

Additionally, food polysaccharides include cellulose, such as fermentation-derived cellulose and microcrystalline cellulose, and modified starch. To meet the diverse market needs for frozen dessert, novel materials with high functionalities are desired.

For example, there has been considered a combined frozen dessert comprising an edible foaming layer containing at least one polysaccharide selected from the group consisting of locust bean gum, guar gum, and xanthan gum, and an ice-cream layer (Patent Literature 3-1).

Patent Literature 3-2 discloses the use of xanthomonas gum (xanthan gum) in ice cream and the like; Patent Literature 3-3 discloses a stabilizer for ice cream containing xanthomonas gum and guar gum; and Patent Literature 3-4 discloses a frozen dessert stabilizer containing xanthomonas gum, locust bean gum, and guar gum. The formulas in these prior art techniques cannot provide the desired effects, and cannot produce a frozen dessert with excellent heat-shock resistance.

Additionally, the Applicant of the present application also discloses, as a means for imparting heat-shock resistance to frozen desserts, a method for incorporating one member selected from tamarind seed polysaccharides, locust bean gum, guar gum, and carrageenan as a stabilizer for a frozen dessert that has a cotton candy-like texture containing a protein hydrolysate derived from wheat as a foaming agent, with the overrun at 100 to 300% (Patent Literature 3-5). The Applicant also discloses in Patent Literature 3-6 a frozen dessert with improved heat-shock resistance that contains locust bean gum and tamarind seed polysaccharide.

Welan gum, produced by microorganisms, is known for its applicability to ink compositions, concrete or cement-based materials, materials for unvulcanized rubber, and cosmetic compositions. Examples of applications being considered include an ink composition for water-based ballpoint pens (Patent Literature 3-7), cement or concrete compositions containing welan gum (Patent Literature 3-8 and Patent Literature 3-9), a parting agent composition for unvulcanized rubber (Patent Literature 3-10), and a cosmetic composition containing gellan gum or its derivative, a solid compound, and an univalent salt (Patent Literature 3-11).

However, improvement and modification of the physical properties and functionality of food that are achieved by using welan gum therein currently have not practically been researched.

Citation List

Patent Literature

Patent Literature 1-1: US Patent No. 3806605
Patent Literature 1-2: EP Patent No. 354356
Patent Literature 1-3: JPH07-236443A
Patent Literature 1-4: Japan Patent No. H07-108201
Patent Literature 1-5: JP2005-295841A
Patent Literature 1-6: JPH04-356160A
Patent Literature 1-7: JPH10-295339A
Patent Literature 1-8: JPH11-56244A
Patent Literature 1-9: JP2000-60507A
Patent Literature 1-10: JP2000-157232A
Patent Literature 1-11: JPS61-67467A
Patent Literature 1-12: JP2004-16148A
Patent Literature 1-13: JP2010-508038A
Patent Literature 1-14: JPH07-252494A
Patent Literature 1-15: JP2011-57747A
Patent Literature 1-16: JP2003-73229A
Patent Literature 1-17: JPH09-143498A
Patent Literature 1-18: JP2011-225763A
Patent Literature 2-1: JPH11-148041A
Patent Literature 2-2: JPS63-315547A
Patent Literature 2-3: JPH06-55529A
Patent Literature 2-4: JP2009-249533A
Patent Literature 2-5: JP2009-298809A
Patent Literature 3-1: JPS62-190050A
Patent Literature 3-2: Japan Patent No. S44-10149
Patent Literature 3-3: JPS52-114054A
Patent Literature 3-4: Japan Patent No. S57-37306
Patent Literature 3-5: JP2002-360178A
Patent Literature 3-6: JP2002-253126A
Patent Literature 3-7: JPH11-148041A
Patent Literature 3-8: JPS63-315547A
Patent Literature 3-9: JPH06-55529A
Patent Literature 3-10: JP2009-249533A
Patent Literature 3-11: JP2009-298809A

Non-patent Literature

Non-patent Literature 1-1: Shokuhintatorui Nyuka, Zonen, Geruka No Chishiki (Food Polysaccharides, Knowledge of Emulsification, Thickening, and Gel Formation), Naomichi Okazaki, Masao Sano, Saiwai Shobo (2001)

Summary of Invention

Technical Problem

An object of the present invention is to provide a method for improving or maintaining physical properties of a substance (in particular, a composition) (e.g., food) (specifically, (1) the retention and stability of foam, (2) the texture of baked foods, and (3) the stability of frozen desserts). More specifically, the present invention has the following objectives.

Given the circumstances stated regarding the technical field 1, an object of the present invention is to improve the retainability and stability of bubbles in foam (air foam)-containing food. Specifically, an object of the present invention is to provide a method for stabilizing foam for example, in foam-containing compositions including foods, such as whipped cream, whipped desserts, and foam-containing beverages; washing agents, such as hair shampoo, body shampoo, body soap, hand soap, facial cleanser, bathroom cleaner, cleanser for exhaust fans, glass cleanser, kitchen detergent, and laundry detergent; and hair dye. Another object is to provide a method for stably retaining foam formed inside the foam-containing composition (e.g., preferably foods, in particular beverages) by increasing the retainability of foam of the foam-containing composition.

Given the circumstances stated regarding the technical field 2, another object of the present invention is to provide a method for improving the texture of edible compositions, such as foods, in particular, baked foods made from egg whites (a raw material) (egg white-containing baked confections), such as baked meringue, by using welan gum.

Given the circumstances stated regarding the technical field 3, another object of the present invention is to improve the stability of frozen desserts, which has not been sufficiently improved with the prior art techniques.

Solution to Problem

The present inventors found that the use of welan gum can solve the problems described above. Thus the inventors completed the present invention.

The outline of the problems of the technical fields 1 to 3 is described below respectively.

Regarding the technical field 1, the present inventors conducted extensive research to solve the problems, and found that the use of welan gum as a foam stabilizer exhibits an effect for improving foam stability in whipped cream, whipped desserts, and foam-containing beverages as described above. The present invention was completed on the basis of these findings.

One embodiment of the present invention has been developed based on these findings, and includes the following aspects.

[Item 1-1] A foam-containing composition comprising welan gum.

[Item 1-2] The foam-containing composition according to Item 1-1, which is a food or a beverage, a washing agent, or a hair dye.

[Item 1-3] The foam-containing composition according to Item 1-1 or 1-2, wherein the food or beverage is a whipped cream, a whipped dessert, or a foam-containing beverage.

[Item 1-4] The foam-containing composition according to Item 1-1 or 1-2, wherein the washing agent is a hair shampoo, a body shampoo, a body soap, a hand soap, a facial cleanser, a bathroom cleaner, a cleanser for exhaust fans, a glass cleanser, a kitchen detergent, or a laundry detergent.

[Item 1-5] The foam-containing composition according to Item 1-1 to 1-4, wherein the welan gum content is 0.01 to 1 mass%.

[Item 1-6] A method for stabilizing foam, the method comprising adding welan gum to a foam-containing composition.

[Item 1-7] The method for stabilizing foam according to Item 1-6, wherein the amount of the welan gum added is 0.01 to 1 mass%.

[Item 1-8] A stabilizer for a foam-containing composition, the stabilizer comprising welan gum.

Regarding the technical field 2, the present inventors conducted extensive research primarily on the basic properties of welan gum, in consideration of the problems in the prior art, and found that welan gum is superior to conventional food hydrocolloids in improving the texture of egg white-containing baked confections, such as baked meringue. Specifically, the inventors confirmed that a unique texture, which cannot be achieved by conventional food hydrocolloids, can be imparted to a target food by the use of welan gum, and completed the present invention.

Specifically, the present invention relates to a welan gum-containing food with improved texture and a method for improving the texture of egg white-containing baked confections, such as baked meringue, by adding welan gum. More specifically, the present invention relates to a texture-improvement effect achieved by adding welan gum, for example, imparting crunchiness to egg white-containing baked confections, such as baked meringue, giving better perceived meltability in mouth and the like.

One embodiment of the present invention has been developed based on these findings, and includes the following aspects.

[Item 2-1] An egg white-containing baked confection comprising welan gum.

[Item 2-2] The egg white-containing baked confection according to Item 2-1, which has an improved texture compared with another egg white-containing baked confection that has been made from the same raw material except that does not contain welan gum.

[Item 2-3] The egg white-containing baked confection according to Item 2-2, wherein the improved texture comprises (1) better crunchiness, (2) better perceived meltability in mouth, or both.

[Item 2-4] A method for producing the egg white-containing baked confection according to any one of Items 2-1 to 2-3, the method comprising a baking process of materials including an egg white and foam.

[Item 2-5] The egg white-containing baked confection according to any one of Items 2-1 to 2-3, produced by the production method according to Item 2-4.

[Item 2-6] A method for improving a texture of an egg white-containing baked confection, the method comprising adding welan gum.

[Item 2-7] The method for improving a texture according to Item 2-6, wherein the improved texture comprises (1) better crunchiness, (2) better perceived meltability in mouth, or both.

[Item 2-8] A method for producing an egg white-containing baked confection, the method comprising adding welan gum.

[Item 2-9] The production method according to Item 2-8, wherein the baked confection has an improved texture compared with an egg white-containing baked confection that has been made from the same raw material except that does not contain welan gum.

[Item 2-10] The production method according to Item 2-9, wherein the improved texture comprises (1) better crunchiness, (2) better perceived meltability in mouth, or both.

[Item 2-11] A texture-improving agent for an egg white-containing baked confection, the improving agent comprising welan gum.

Regarding the technical field 3, the present inventors conducted extensive research to solve the problems described above, and found that adding welan gum to a frozen dessert improves the stability of the frozen dessert. Specifically, the dispersoids contained in a frozen dessert, such as solid components, foam, and ice, were homogeneously dispersed in the dispersant, by adding welan gum to the frozen dessert; and the desired condition of the frozen dessert was maintained. The inventors found that adding welan gum can improve the dispersibility of the solid components and the like contained in a frozen dessert, the formability and shape retainability of the frozen dessert, the heat-shock resistance, the whipping ability, the scoopability, the perceived meltability in mouth, and the like.

One embodiment of the present invention has been developed based on these findings, and includes the following aspects.

[Item 3-1] A frozen dessert comprising welan gum (preferably, a frozen dessert comprising welan gum and having high stability).

[Item 3-2] The frozen dessert according to Item 3-1, comprising the welan gum in an amount of 0.001 to 1.0 mass%.

[Item 3-3] The frozen dessert according to Item 3-1 or 3-2, further comprising locust bean gum and/or guar gum.

[Item 3-4] The frozen dessert according to Item 3-3, further comprising tamarind seed gum.

[Item 3-5] A method for stabilizing a frozen dessert, the method comprising adding welan gum.

[Item 3-6] The method for stabilizing a frozen dessert according to Item 3-5, wherein the welan gum is added to the frozen dessert in an amount of 0.001 to 1.0 mass%.

[Item 3-7] The method for stabilizing a frozen dessert according to Item 3-5 or 3-6, further comprising adding locust bean gum and/or guar gum.

[Item 3-8] The method for stabilizing a frozen dessert according to Item 3-7, further comprising adding tamarind seed gum.

[Item 3-9] A method for producing a frozen dessert, the method comprising adding welan gum.

[Item 3-10] The method for producing a frozen dessert according to Item 3-9, wherein the welan gum is added to the frozen dessert in an amount of 0.001 to 1.0 mass%.

[Item 3-11] The method for producing a frozen dessert according to Item 3-9 or 3-10, further comprising adding locust bean gum and/or guar gum.

[Item 3-12] The method for producing a frozen dessert according to Item 3-11, further comprising adding tamarind seed gum.

Advantageous Effects of Invention

An embodiment of the present invention can improve the retention and stability of foam in a foam-containing food.

Additionally, an embodiment of the present invention can improve the texture of an egg white-containing baked confection. In particular, an embodiment of the present invention can provide an egg white-containing baked confection that has improved texture compared with conventional egg white-containing baked confections. More specifically, with addition of welan gum, texture of an egg white-containing baked confection can be improved, for example, by imparting better crunchiness or better perceived meltability in mouth.

Additionally, an embodiment of the present invention can improve the stability of a frozen dessert. In particular, an embodiment of the present invention can provide a frozen dessert that exhibits a homogeneous dispersion state, improved heat-shock resistance (effects of suppressing syneresis and leakage of sugar), and also whipping ability, texture, formability, shape retainability, scoopability, or perceived meltability in mouth.

Brief Description of Drawings

Fig. 1 is a graph illustrating changes in Brix of meltwater from frozen beverages with a pH of 2.6 prepared in Test Example 3-7.

Fig. 2 is a graph illustrating changes in Brix of meltwater from frozen beverages with a pH of 3.1 prepared in Test Example 3-7.

Fig. 3 is a graph illustrating changes in Brix of meltwater from frozen beverages with a pH of 3.6 prepared in Test Example 3-7.

Description of Embodiments

Terms

Unless otherwise specified, the symbols and abbreviations used in the present specification can be understood to have the meanings generally used in the technical field to which the present invention pertains, in accordance with the context of the present specification.

In the present specification, the term "comprise" is used with the intention of including the terms "consist essentially of" and "consist of."

Unless otherwise specified, the step, treatment, and operation described in this specification can be performed at room temperature.

In the present specification, the room temperature is a temperature between 10°C and 40°C.

In the present specification, the "food" refers to various substances that are processed, semi-processed, or unprocessed, intended for human consumption.

In the present specification, the "processed food product" refers to food that is a processed substance.

In the present specification, the material composition of the "processed food product" (i.e., a composition for food processing) can be a semi-processed substance, or a composition containing an unprocessed substance.

In the present specification, the "food" includes, in addition to narrowly defined foods, beverages, chewing gums, and all substances used for production, preparation, or processing of foods.

In the present specification, the "food" includes "Food with Health Claims", "Foods with Function Claims", "Foods for Specified Health Uses", "Foods with Nutrient Function Claims", and "Food for Special Dietary Uses".

In the present specification, "adding" may refer to the use (or addition) of a substance as a raw material for a product (including semi-finished products), depending on the context of the present specification.

1. Foam-Containing Composition

The foam-containing composition of the present invention comprises welan gum, which imparts a high foam stability.

The foam-containing composition preferably exhibits suitable properties for its use thereby forming foam (or containing foam). Typical examples of such a composition include cleansers that exhibit their cleansing effect by retaining a large amount of foam. However, this description is just for an explanation, and does not limit the present invention.

The amount of foam and the state of foam in the foam-containing composition are not particularly limited, and the composition can be, for example, those retaining foam in at least one portion (e.g., the upper surface, the upper portion, the upper layer, and the inner portion), or those that are entirely in the form of foam.

The foam-containing food, which is an embodiment of the foam-containing composition of the present invention, includes narrowly defined foods (e.g., of foods in a broad sense, those that can be consumed by eating) and beverages (e.g., of foods in a broad sense, those that can be consumed by drinking).

The foam-containing food of the present invention refers to foods that contain foam, and that can be eaten or drunk together with the foam. The amount of the foam in the food is not particularly limited, and the food may be any of a food retaining foam in its upper surface, a food some portion of which changes into foam, or a food that entirely changes into foam.

Specific examples include foods, such as whipped desserts, including whipped cream, fresh cream, mousse, and marshmallow; foam-containing beverages, such as smoothie-like beverages, cappuccino, and milkshakes; confectionery, such as meringue, chocolate, and gummi candy; and frozen desserts, such as ice cream, ice milk, and lacto-ice.

Another example of the foam-containing composition of the present invention includes washing agents. In particular, preferable washing agents include those for body or households (e.g., washing agents used for washing body surfaces, such as hair shampoo or body shampoo, facial cleansers, and hand soap; and washing agents used for floors, walls, windows, bathrooms (in particular, the bathtubs), washstands, or toilets of homes).

Another example of the foam-containing composition of the present invention includes hair dyes for silver hair, hair dyes for coloring, cosmetics, shaving foam, effervescent bath additives (bath fizz), hair removers, hair-growth stimulants, pesticides, bleaching agents, extinguishers, feeds for ornamental-fish, garden fertilizers, and gloss agents for automobile tires or leather shoes.

These may be preferably compositions that can exhibit properties suitable for the use thereof, by forming foam (or containing foam).

The technique of the present invention can even be applied to foam-containing compositions other than the examples described above, as long as the compositions can suitably contain a large amount of foam.

The foam stabilizer of the present invention comprises welan gum. Welan gum primarily comprises a polysaccharide obtained from a liquid culture medium of a *Sphingomonas* bacterium (*Sphingomonas sp.*). For convenience, commonly distributed commercial products can be used, and specific examples include VIS TOP W of San-Ei Gen F.F.I., Inc.

The use of welan gum as a foam stabilizer of a foam-containing composition improves the stability of the foam (an object of the present invention).

Additionally, the separation and aggregation of components of the foam-containing composition caused by long-term storage or storage at room temperature or higher in the form of the liquid starting material can also be decreased significantly decreased. Thus, the liquid starting material before introducing foam can be stored for a long time at room temperature maintaining its quality to a certain level. Additionally, even an existing liquid starting material that do not contain welan gum can also exhibit equivalent foam stability by introducing foam after adding welan gum.

The amount of welan gum contained in the foam-containing composition is not particularly limited as long as the effect of the present invention can be provided, and the amount can suitably be adjusted depending on the type of the composition or the proportion of each component in the composition.

Specifically, when the foam-containing composition is a food (e.g., whipped cream and a whipped dessert), welan gum can be contained in such a food in an amount of 0.01 to 1 mass%, and preferably 0.05 to 0.5 mass%, per 100 mass% of the food.

When the foam-containing composition is a beverage (e.g., a foam-containing beverage), welan gum can be contained in such a beverage in an amount of 0.01 to 1 mass%, and preferably 0.05 to 0.3 mass%, per 100 mass% of the beverage.

When the foam-containing composition is a washing agent, welan gum can be contained in such a washing agent in an amount of 0.01 to 1 mass%, and preferably 0.02 to 0.8 mass%, per 100 mass% of the washing agent.

The amount of welan gum added is, however, not limited to the above-mentioned ranges, and can be suitably adjusted depending on the composition to which welan gum is added.

The effects of the present invention are provided by producing the foam-containing composition of the present invention in accordance with an ordinary method using welan gum, which is the foam stabilizer according to the present invention, or is an active constituent thereof, as one ingredient of the composition.

For example, when preparing a foam-containing coffee with the foam stabilizer according to the present invention, the coffee may be prepared using welan gum as a part of the ingredients of the coffee beverage in accordance with an ordinary method. In this case, the foam of the coffee beverage do not being formed when the coffee has been prepared; however, foam can be formed by shaking the container before drinking.

The foam-containing composition of the present invention may contain, in addition to the welan gum, another polysaccharide thickener applicable to the production of the foam-containing composition.

The polysaccharide thickener is not particularly limited, and any thickener can be used as long as the thickener does not significantly hinder the effects of the welan gum on the foam-containing composition.

Specifically, such a polysaccharide thickener includes carrageenan, glucomannan, gum arabic, gum ghatti, gellan gum, guar gum, locust bean gum, xanthan gum, pectin, alginic acid and its salts, soybean soluble polysaccharides, psyllium seed gum, tara gum, gum karaya, gum tragacanth, tamarind seed gum, fermented cellulose, microcrystalline cellulose, carboxy methylcellulose, curdlan, pullulan, and starch. These polysaccharide thickeners may be used singly, or in a combination of two or more.

The content of welan gum and the content of a polysaccharide thickener in the foam stabilizer of the present invention may vary depending on the types and amounts of components contained, as well as desired properties (e.g., the texture) of the foam-containing composition (e.g., food) produced, and can be suitably adjusted and determined.

Various components typically used in preparing foam-containing compositions may be used in the foam-containing composition of the present invention to the degree that the effects of the present invention are not significantly hindered.

When the foam-containing composition of the present invention is a food, examples of various components include dairy products, sugar, emulsifiers, flavors, and fats and oils.

The dairy products, emulsifiers, and fats and oils each include a wide range of those typically used in preparing whipped cream, whipped desserts, and the like.

Specifically, examples of dairy products include cow milk, fresh cream, skimmed milk powder, concentrated milk, and condensed milk; and examples of fats and oils include butter, coconut oil, palm oil, and rapeseed oil. However, dairy products and fats and oils are not limited to these examples.

Additionally, as other components, food ingredients, such as proteins, dietary fibers, and seasonings are listed.

For instance, when the foam-containing composition targeted by the present invention is a washing agent, examples of other components include soap, such as bar soap, powdered soap, and mixture of soap with synthetic detergent; surfactants, such as anionic surfactants (e.g., linear alkyl benzene sulfonate, alkylsulfuric acid ester salts, α-olefin sulfonic acid salts, ether sulfuric acid ester salts, alkyl phenyl polyethylene glycol ethers, alkyl polyethylene glycol ethers), nonionic surfactants (e.g., polyoxyethylene alkyl ethers, fatty acid alkanolamides, and alkyl polyglucosides), cationic surfactants (e.g., long-chain dialkyl quaternized ammonium salts and long chain monoalkyl quaternized ammonium salts), and ampholytic surfactants (e.g., alkyl dimethyl aminoacetate betaine, and fatty acid amidopropyl betaine); detergent builders, such as inorganic builders (e.g., various zeolites, amorphous aluminosilicate, sodium pyrophosphate, sodium carbonate, crystalline sodium silicate, sodium sulfate, and sodium tripolyphosphate), and organic builders (e.g., aminocarboxylate, hydroxy aminocarboxylate, hydroxycarboxylate, cyclocarboxylate, ether carboxylate, and polycarboxylates); alkaline agents, such as carbonates, silicates, phosphates, borates, and amines; enzyme preparations, such as protease, lipase, and amylase; anti-redeposition agents, such as carboxy methylcellulose (CMC), and polyethylene glycol; and others, such as bleaching agents, softening agents, foam control agents, optical brighteners, reducing agents, flavors, dyes, and antimicrobial agents.

When the foam-containing composition of the present invention is a gloss agent for hair dyes or automobile tires as well, components commonly used in these products can be used with no restriction.

The method for forming foam is not particularly limited, and any foaming method can be used according to the type of the foam-containing composition.
For example, the following methods may be used:

(1) a method for forming foam with a stirrer, such as an industrial mixer, a beater, or a cutting machine, an instrument, such as a whisk or a handheld mixer, as well as a squeeze foamer, a foam pump, and containers, such as a spray container, an aerosol container, or a non-aerosol container that have been conventionally used in forming foam;
(2) a method in which the whole container containing a foam-containing composition is shaken; and
(3) a method for forming foam of carbon dioxide by mixing with water.

These methods may be used singly, or in a combination of two or more.

Examples of the method (3) include a method in which acids and carbonates are mixed simultaneously or individually with an aqueous solvent. The aqueous solvent is not particularly limited as long as the solvent contains water; examples include water, boiled water, cow milk, soybean milk, fruit-juice beverages, and chocolate beverages.

The type of the acid is not particularly limited, and an organic acid or an inorganic acid may be used.

Examples of the organic acid include tartaric acid, citric acid, fumaric acid, succinic acid, malic acid, gluconic acid, oxalic acid, malonic acid, glutaric acid, adipic acid, glycolic acid, diglycolic acid, nitrilotriacetic acid, ethylenediaminetetraacetic acid, ascorbic acid, lactic acid, and acetic acid, as well as sodium salts or potassium salts thereof. Examples of the inorganic acid include phosphates (e.g., tricalcium phosphate, calcium monohydrogen phosphate, disodium hydrogen phosphate, dipotassium hydrogen phosphate, calcium dihydrogen phosphate, sodium dihydrogen phosphate, potassium dihydrogen phosphate, tetrapotassium pyrophosphate, sodium metaphosphate, potassium metaphosphate, sodium polyphosphate, and potassium polyphosphate).

In particular, the acid is preferably at least one member selected from organic acids, and more preferably at least one member selected from the group consisting of tartaric acid, citric acid, fumaric acid, succinic acid, malic acid, and gluconic acid.

The type of the carbonate is not particularly limited, and the carbonate is, for example, at least one member selected from sodium carbonate, sodium hydrogen carbonate, potassium carbonate, potassium hydrogen carbonate, ammonium carbonate, ammonium hydrogen carbonate, sodium sesquicarbonate, calcium carbonate, or magnesium carbonate. The carbonate is preferably, for example, at least one member selected from sodium carbonate, sodium hydrogen carbonate, potassium carbonate, potassium hydrogen carbonate, calcium carbonate, or magnesium carbonate; and the carbonate is more preferably, for example, at least one member selected from sodium hydrogen carbonate, sodium carbonate, potassium hydrogen carbonate, or potassium carbonate.

As described above, the present invention can improve the stability of the foam-containing composition, and can also extend the time period from the foam generation to the foam collapse (i.e., the retention time of foam), increase the foam density, and/or suppress the syneresis.

When the foam-containing composition of the present invention is a washing agent, the dense foam can be retained for a long time without foam collapse, and thereby sufficiently contact with stained portions of the object to be washed. As a result, for example, the surfactant and the component having an antimicrobial or sterilizing effect contained in the washing agent can be in contact with the stained portions for a long time, and can maximize their effect. Because of this, a washing agent can exhibit a sufficient washing effect even in a small amount, and thereby reduces the problems such as detergent residues or soap residues.

The washing agent also, together with foam, clings to the object; this can suppress the drop-off of foam when the agent is sprayed onto the surface of the object to be washed. The washing agent is thus easier to use than a liquid washing agent, and is also usable for angled surface of objects or an intricately shaped object for which liquid washing agents cannot be used, because such liquid agents flow down.

When the foam-containing composition is a shower gel, it is possible to obtain effects such as improving the dense and creamy texture (the full-bodied texture) when it touches the skin.

When the foam-containing composition is a hair dye, its dense foam do not drop off and spread all over the scalp hair, thereby uniformly coming in contact with scalp hair.

When the foam-containing composition is a hair remover or hair-growth stimulant, the time period during which the composition adheres to the scalp surface is extended, improving the effect of the active component contained in the product.

When the foam-containing composition is an extinguisher, the foam can extensively cover the fire origin for a long time, blocking the oxygen supply and thereby efficiently extinguishing the fire.

When the foam-containing composition is a feed for ornamental fish, the ornamental fish eats floating foam on the water surface. This improves the convenience of feeding and the visibility because of the striking appearance.

When the foam-containing composition is a garden fertilizer, the fertilizer component is expected to gradually be released through over a long period of time. Because the composition has foam appearance, the fertilizer is also excellent from an aesthetic perspective.

When the foam-containing composition is a gloss agent for automobile tires, fine foam uniformly adhere to the tire surface for a long time, lustering the tire without uneven coating.

"The method for stabilizing foam" of the present invention and "the stabilizer for a foam-containing composition" of the present invention" are both understood from the description regarding "the foam-containing composition" and the like. In particular, "the stabilizer for a foam-containing composition" can preferably be a foam stabilizer .

2. Egg White-Containing Baked Confection

The egg white-containing baked confection targeted by the present invention is a food obtained by performing a foaming step (e.g., a step of whipping egg whites) and then performing a baking step. Specific examples include baked confections, such as baked meringue and macaroons.

The order of adding welan gum to these foods is not particularly limited, and welan gum may be added before whipping egg whites or during the whipping of egg whites; or added and mixed after whipping egg whites.

In the egg white-containing baked confection with an improved texture and the method for improving the texture of the confection according to the present invention, it is preferable to add welan gum such that welan gum is present in an amount of preferably 0.001 to 1 wt% in a food on a before-baking-weight basis. With lower concentration than 0.001 wt%, welan gum cannot exhibit a sufficient texture-improving effect. Conversely, with higher concentration than 1 wt%, welan gum cannot provide any further effect; instead, the production or handling efficiency is reduced due to increased viscosity.

In the egg white-containing baked confection with an improved texture and the method for improving the texture of the confection according to the present invention, a polysaccharide thickener other than welan gum can be added as long as the effects of welan gum are not significantly hindered. In addition, optional components may also be added, such as a starch, an emulsifier, a seasoning, a flavor, a color additive, a sweetener, an acidulant, a baking powder, a skimmed milk powder, a shelf-life extender, a preservative, and an antioxidant. The addition of a polysaccharide thickener enhances the texture achieved by welan gum, and is thus useful in preparing an egg white-containing baked confection with a lighter and crunchier texture and excellent perceived meltability in mouth. A baking powder can be used as a foaming agent for preparing foam in the present invention.

The polysaccharide thickener include guar gum, gelatin, gellan gum, carrageenan, xanthan gum, locust bean gum, glucomannan, agar, alginic acid, sodium alginate, tamarind seed gum, tara gum, gum arabic, gum tragacanth, gum karaya, pectin, pullulan, carboxy methylcellulose or its sodium salt, microcrystalline cellulose, fermentation-derived cellulose, and furcellaran. These may be used singly, or in a combination of two or more.

Additionally, welan gum in the present invention may be formulated with food additives etc. used for egg white-containing baked confections. Welan gum formulated into a texture-improving agent has an advantage in improvement of the convenience during the production steps of the egg white-containing baked confection.

Components that can be formulated in the texture-improving agent together with welan gum include food additives and food materials used for egg white-containing baked confections without limitation, as described above.

Specific examples include thickening stabilizers (e.g., xanthan gum, galactomannan, gellan gum, carrageenan, cellulose, soybean soluble polysaccharides, starches (e.g., modified starches), and gelatin); emulsifiers (e.g., lecithin, sucrose fatty acid ester, and glycerin fatty acid ester); and bulking agents (e.g., dextrin, cyclodextrin, glucose, sucrose, lactose, and trehalose).

The proportion of each component of the texture-improving agent can be determined as any amount and proportion depending on the egg white-containing baked confection produced.

The form of welan gum added to an egg white-containing baked confection in the present invention is not limited as long as the form is used in food production. Welan gum may be in the form of a texture-improving agent as described above, or welan gum may be added in any form, such as powder, solution, or paste.

The baked confection of the present invention has an improved texture compared with egg white-containing baked confections that have been made from the same ingredients but that do not contain welan gum. The improved texture comprises (1) better crunchiness, (2) better perceived meltability in mouth, or both. Additionally, the occurrence of uneven baking is reduced. These are presumably based on the uniform dispersion of bubbles in a food and smaller variations in the size of bubbles due to the addition of welan gum; however, the present invention is not bound by this theory.

The texture improving effect achieved by the present invention includes imparting a light and crunchy texture, and better perceived meltabiliry in mouth after chewing, to egg white-containing baked confections, such as baked meringue and macaroons.

The egg white-containing baked confection of the present invention can be produced by a typical method, except for the use of welan gum. The baking can also be performed by a typical method under ordinary conditions. In the method for producing the egg white-containing baked confection of the present invention, welan gum can simply be added as a part of the ingredients of the food, and the method for adding welan gum to the food is not particularly limited. A known method can be used, for example, a method in which welan gum is mixed with other powdery ingredients beforehand, a method in which welan gum is dissolved in water beforehand and then added, and a method in which welan gum is mixed by kneading. Additionally, because any particular equipment and conditions are not required in the production, the present invention has economic advantages.

The present invention also relates to the egg white-containing baked confection with an improved texture that comprises welan gum, and the method for improving the texture of an egg white-containing baked confection in which welan gum is added.

"The texture-improving agent for an egg white-containing baked confection" of the present invention can be understood from the description regarding "the egg white-containing baked confection" and the like.

3. Frozen Dessert

The frozen dessert of the present invention can be in various forms, depending on the intended product. Examples include ice cream (e.g., ice cream, lacto-ice, and ice milk), soft-serve ice cream, freezed cream cake, cracker ice cream sandwiches, antifreeze cream cake, ice cream in a cone, ice cream in a cup, choux pastry filled with ice cream, *monaka* waffle filled with ice cream, soft-serve ice cream mix, ice cream mix, sherbets, ice pops, *mizore* ice, shaved ice, frozen yogurts, frozen beverages, and shakes.

In the present invention, the amount of welan gum added to a frozen dessert can be suitably adjusted, depending on the intended use (an effect imparted to the frozen dessert) of welan gum or the ingredients. For example, to impart a smooth texture and heat-shock resistance (a syneresis suppression effect) to a frozen dessert, it is preferable to add welan gum typically in an amount of 0.001 to 1.0 mass%, and preferably 0.01 to 0.5 mass%, based on the frozen dessert.

If the amount of welan gum is lower than 0.001 mass%, a sufficient texture and heat-shock resistance (a syneresis suppression effect) cannot be achieved. On the other hand, if the amount of welan gum is 1.0 mass% or more, any further effect cannot be achieved, instead reducing the production or handling efficiency due to increased viscosity and the like.

To improve the dispersibility of solids, it is preferable to add welan gum in an amount of typically 0.01 to 1.0 mass%, and preferably 0.02 to 0.8 mass% based on the frozen dessert.

If the amount of welan gum is lower than 0.01 mass%, a sufficient dispersibility cannot be achieved. Conversely, if the amount of welan gum is 1.0 mass% or more, any further effect cannot be achieved, instead reducing the production or handling efficiency due to increased viscosity and the like.

The addition of welan gum to the frozen dessert of the present invention may be performed by adding welan gum alone, or by forming a preparation of welan gum in conbination with various components conventionally used in the production of frozen desserts described later, and adding it as a stabilizer for frozen desserts.

The welan gum content in the stabilizer for frozen desserts can suitably be adjusted such that the amount of welan gum added falls within the ranges described above.

Specifically, in the present invention, the addition of welan gum or the stabilizer for a frozen dessert comprising welan gum of the present invention to a frozen dessert leads to homogeneous dispersion of dispersoids contained in the frozen dessert, such as solid components, foam, and ice, in a dispersant, maintaining the state immediately after the frozen dessert has been prepared. Because of this physical function of welan gum on the frozen dessert, the present invention can provide the following effects:

(1) improve the formability or shape retainability of the frozen dessert;

(2) reduce the leakage of sugar and syneresis, thus improving the heat-shock resistance of the frozen dessert;
(3) improve the dispersibility of insoluble solids, such as pulps in fruit juice, and solid components, such as frozen moisture by formed by freezing and crushed ice;
(4) homogeneously disperse bubbles, thus improving overrun;
(5) improve scoopability or perceived meltability in mouth; and
(6) make it easier to squeeze out a frozen beverage.

In the present invention, "stabilization" means to achieve at least one effect of the effects described above.

In the present invention, "having high stability" means that at least one effect of the effects described above has been achieved.

The effects (2) and (3) of the present invention relate to imparting heat-shock resistance achieved by a sugar migration suppression effect and a syneresis suppression effect.

Typically, frozen desserts may melt by opening and closing of the freezer, or temperature change during storage and distribution (heat shock). This results in occurrence of the syneresis phenomenon, in which the moisture leaks out from frozen desserts, and the phenomenon in which the sugar leaks out from frozen desserts.

A structure that undergoes greater syneresis when melting refers to a structure that exhibits significantly poor stability of water, fat and oil (these can be solids), and/or air contained in the structure. In such an unstable structure, components are likely to migrate during heat shock in a container, leading to problems such as uneven structure, uneven taste, changes in texture, evaporation of moisture developed by syneresis, shrinkage of frozen desserts (a decrease in volume) due to leakage of air captured in the frozen dessert, spoilage of the crunchy or crispy texture of freshly baked cones or *monaka* waffles due to the moistening effect caused by syneresis or volatilization of ice cream, and the like. The syneresis suppression effect of the present invention can decrease the spoilage of the cone texture of ice cream and the texture of thin crisp wafers of *monaka* ice cream, and thus frozen desserts that exhibit excellent long-term stability can be provided.

The syneresis rate of the frozen dessert of the present invention can be 7% or less, for example, when allowed to stand on filter paper at room temperature (25°C) for 60 minutes.

The phenomenon such that the sugar leaks out often occurs, in particular, in fat-free frozen desserts, such as ice pops or sherbets. This is the problem such that the sugar migrates during heat shock, and causes uneven distribution, thus leading to uneven taste, changes in the texture, and adhesion to package caused by the leaked sugar. With the stabilizer for a frozen dessert of the present invention, a significantly stable structure can be formed, which reduces the occurrence of syneresis when a frozen dessert is melting, and reduces sugar leak; accordingly, during heat shock, the components in a frozen dessert are prevented from becoming uneven, and the frozen dessert is prevented from shrinking and adhering to the package, thus heat-shock resistance can be imparted to the frozen dessert.

Moreover, the addition of welan gum to a frozen dessert improves the shape formability of the frozen dessert. Thus the sharper edges can be shaped in the serving process of the soft-serve ice, which is prepared by squeezing softened ice cream after freezing out into a cone to form a cone-shape unique to soft-serve ice cream, followed by hardening, and its shape can be maintained until the soft-serve ice cream is completely hardened to the degree at which the ice cream is distributed or sold. This effect is useful not only in squeezing out ice cream into a cone, but also for frozen desserts obtained by sandwiching ice cream by food materials, such as *monaka* waffles or biscuits. When ice cream is squeezed out into a *monaka* waffle, a biscuit, or the like, the ice cream does not easily lose its shape. Thus, a sufficient amount of ice cream can be uniformly placed between *monaka* waffles or a biscuits. Moreover, when a food material other than ice cream, such as cooled sweet bean paste, sauce, or chocolate (e.g., in the form of solid or fluid, such as chocolate sauce), is further placed between *monaka* waffles, the shape of ice cream can also be maintained. Moreover, when a *monaka* waffle or biscuit that serves as a lid is placed from above, the shape of ice cream can also be maintained. Thus, a frozen dessert with a preferable shape as a sandwich-type frozen dessert can be produced. In summary, the present invention has excellent effects on frozen dessert production.

The frozen dessert of the present invention can be produced by adding welan gum described above in a typical production step of frozen desserts. For example, the frozen dessert can be produced by suitably selecting a necessary step from weighing and mixing of ingredients -> heating (30 to 80°C) - > mixing and dissolving -> filtration -> homogenization -> sterilization (sterilization at 68°C for 30 minute or more; HTST sterilization; or UHT sterilization) -> cooling (5°C or below) -> aging > adding flavor -> freezing -> packing -> hardening. The stabilizer for a frozen dessert of the present invention can typically be weighed and mixed with other ingredients in the step of weighing and mixing ingredients.

It is preferable for the frozen dessert of the present invention that the soluble solids content is set within the range of 1 to 60 mass%, and more preferably 10 to 40 mass%. Components of the soluble solids are not particularly limited as long as they are water-soluble solids that are typically used in frozen desserts, and may be the same as such components of typical frozen desserts. Specifically, as long as the effects of the present invention are provided, at least one ingredient selected from the group consisting of water, fats and oils, fruit juice, proteins, sweeteners, nonfat milk solids, flavorings, acidulants, color additives, emulsifiers, and antioxidants, other than the welan gum described above, can be mixed and dissolved in predetermined proportions for use.

For the fat and oil, one member selected from vegetable fats and oils, milk fat such as butter, fractionated fats and oils thereof, hydrogenated fats and oils thereof, and transesterified fats and oils thereof may be used singly, or these may be used in a combination of two or more. Examples of vegetable fats and oils include coconut oil, palm oil, soybean oil, canola oil, cottonseed oil, corn oil, sunflower oil, olive oil, safflower oil, cacao butter, and palm kernel oil.

For the protein, typically, components containing milk-derived protein, such as cow milk, skimmed milk powder, whole milk powder, whole-milk sweetened condensed milk, sweetened condensed skimmed milk, condensed skimmed milk, or fresh cream; or egg-derived protein, such as egg whites, are suitably used.

Examples of the sweetener include sugar, such as sucrose, isomerized sugar, lactose, maltose, glucose, fructose, invert sugar, starch syrup, corn syrup solids, maltitol, honey, trehalose, palatinose, and D-xylose; sugar alcohols, such as xylitol, sorbitol, maltitol, and erythritol; and high-intensity sweeteners, such as sodium saccharine, cyclamate and its salt, acesulfame potassium, thaumatin, aspartame, sucralose, alitame, neotame, advantame, and stevioside contained in stevia extracts.

For the emulsifier, emulsifiers typically used in frozen desserts may be used. Examples include glycerin fatty acid esters (e.g., monoglycerin fatty acid ester, diglycerin fatty acid ester, organic acid monoglycerides of citric acid, lactic acid, or the like, polyglycerin fatty acid ester), sucrose fatty acid esters, sorbitan fatty acid esters, propylene glycol fatty acid esters, lecithin, saponins, polysorbates, and stearoyl lactate (sodium salts, calcium salts).

For the stabilizer for a frozen dessert of the present invention, other polysaccharides and the like may be used in combination as long as the effects of welan gum are not significantly hindered. Other polysaccharides are, for example, one member or two or more selected from glucomannan, galactomannan (e.g., locust bean gum, guar gum, and tara gum), tamarind seed gum, carrageenan, gum tragacanth, gum karaya, xanthan gum, deacylated gellan gum, native gellan gum, gum arabic, gum ghatti, macrophomopsis gum, agar, gelatin, pectin, curdlan, alginic acids (e.g., alginic acid and alginates), carboxy methylcellulose, methylcellulose, hydroxypropylcellulose, hydroxypropyl methylcellulose, microcrystalline cellulose, fermentation-derived cellulose, microfibrillated cellulose, soybean soluble polysaccharides, hydrolyzed gluten, and starches with interfacial activity.

Additionally, flavors and color additives for use in frozen desserts can be selected and used without limitations. Moreover, food ingredients used for nutritional enrichment, such as minerals (e.g., calcium), vitamins, catechins, and proteins; and insoluble solids for imparting variations of flavor and texture to frozen desserts, such as fruit flesh, nuts, cookies, chocolate, crouton, bread, and sauces, may be suitably added to the frozen dessert.

The substances (e.g., compositions) (particularly food), agents, methods, and production methods, and the like of the present invention can be understood, taking into consideration common technical knowledge, with reference to the descriptions in the present specification.

Examples

The following describes the present invention in detail with reference to the Examples and Test Examples below. However, the present invention is not limited to these Examples.

In the formulas, the unit of values is "part by mass," unless otherwise specified.

The term "part" refers to "part by mass," unless otherwise specified.

The expression "%" refers to "mass%," unless otherwise specified.

In this specification, *1 indicates a product of San-Ei Gen F.F.I., Inc.

In this specification, *2 indicates a registered trademark of San-Ei Gen F.F.I., Inc.

The evaluation expression "AA" represents "particularly excellent," "A" represents "excellent," "B" represents "good," and "C" represents "poor."

The "blank" in each table indicates a blank for the Examples or Comparative Examples in the table (or in a group in the table).

Test Example 1-1: Acidified Milk Beverage

In accordance with the formula shown in the following Table 1-1, acidified milk beverages were prepared. Table 1-2 shows the type and amount of each foam stabilizer used. Table 1-2 also shows the viscosity, the shaking test evaluation, and the state after storage of the obtained acidified milk beverages.

Table 1-1

| Skimmed Milk Powder | 1.5 |
| --- | --- |
| Sugar | 7.0 |

(continued)

| Sucralose | 0.002 |
|---|---|
| Stabilizer for Acidified Milk Beverage (HM Pectin SM-666*1) | 0.2 |
| Stabilizer for Acidified Milk Beverage (Soybean Soluble Polysaccharide SM-1200*1) | 0.3 |
| Foam Stabilizer | See Table1-2 |
| Trisodium Citrate | 0.02 |
| Citric Acid (Anhydrous) | 0.2 |
| Emulsifier (Maruzen Pharmaceuticals Co. Ltd., Quillajanin C-100) | 0.05 |
| With addition of Ion-Exchanged Water | 1 100.0% |

Preparation Method

Step 1) A 20% skimmed milk powder solution was prepared.

Step 2) A powdery mixture of sugar, a stabilizer for acidfied milk beverages, a foam stabilizer for, and trisodium citrate was added to a container containing ion-exchanged water; and the mixture was dissolved with stirring at 80°C for 10 minutes, and then cooled down to room temperature.

Step 3) Sucralose, the preparation obtained in step 1), citric acid, and an emulsifier were added to the preparation obtained in step 2) in this order, and the total amount was adjusted.

Step 4) After the temperature was increased to 75°C, homogenization was performed (first step: 10 MPa, second step: 5 MPa). When the temperature reached 93°C, the resulting product was hot-packed in a PET container.

Table 1-2

| | Foam Stabilizer | Amount | Viscosity* (mPa·s) | Shaking Test | |
|---|---|---|---|---|---|
| | | | | Immediate After Shaking | After Standing for 10 Minutes |
| Blank | - | - | 9.5 | A (88 ml/-) | C (88 ml/57 ml) |
| Comparative Example 1-1 | Guar Gum (VIS TOP*2 D-20*1) | 0.2% | 20.6 | A (82 ml/-) | C (82 ml/58 ml) |
| Comparative Example 1-2 | Fermentation-derived cellulose blend (SAN ARTIST*2 PG*1) | 0.5% | 220.0 | A (78 ml/-) | B (75 ml/59 ml) |
| Example 1-1 | Welan Gum (VIS TOP*2 W*1) | 0.2% | 175.0 | A (75 ml/-) | A (75 ml/-) |
| * The viscosity measurement condition: B-type rotational viscometer at 5°C, 60 rpm ** Shaking Test: The numerical values in the parentheses indicate "the entire volume/the liquid layer portion volume." | | | | | |

Test Method

Step 1) 60 ml of the sample was placed in a 100-ml graduated cylinder so as not to whip.
Step 2) The cylinder was shaken up and down 30 times, and the state of the sample was observed immediately after shaking, and also observed after the sample was allowed to stand for 10 minutes.

Evaluation Method

A: There is no interface.
B: There is an interface, but it is pale and obscure.
C: There is a clear-cut interface.

Evaluation

Welan gum, guar gum, or fermentation-derived cellulose was added to the acidified milk beverages, and the foamability of the resulting beverage was examined by shaking it. Comparative Example 1-1, to which guar gum was added, exhibited separation of the foam and the liquid layer after the beverage was allowed to stand for 10 minutes. However, Example 1-1, in which welan gum was used, exhibited an effect equivalent to that of the case where fermentation-derived cellulose was used, although the amount of welan gum was smaller than that of fermentation derived cellulose; and a liquid layer was not formed, with the foam stably maintained, even after the beverage was allowed to stand for 10 minutes.

Test Example 1-2: Whipped Dessert

In accordance with the following formula shown in Table 1-3, whipped desserts were prepared. Table 1-4 shows the type and amount of each foam stabilizer used. Table 1-4 also shows the values of the viscosity and overrun of the dessert mixes, and evaluation result of foaming state, and foam retainability of the obtained whipped desserts.

Table 1-3

| | |
|---|---|
| Milk | 40.0 |
| Fresh Cream | 6.0 |
| Coconut Oil | 6.0 |
| Skimmed Milk Powder | 6.0 |
| Sugar | 10.0 |
| Gelatin (SPECIAL GELATIN*1) | 1.8 |
| Foam Stabilizer | See Table 1-4 |
| Emulsifier (HOMOGEN*2 No. 897(F)*1) | 0.2 |
| Trisodium Citrate | 0.1 |
| Flavor (VANILLA FLAVOR No. 93-I*1) | 0.1 |
| With addition of Ion-Exchanged Water | 100.0% |

Preparation Method

Step 1) While ion-exchanged water, cow milk, fresh cream, and coconut oil were stirred, skimmed milk powder, sugar, gelatin, a foam stabilizer, the emulsifier, and trisodium citrate were added thereto, followed by stirring under heating for dissolution at 80°C for 10 minutes.

Step 2) The flavor was added thereto, and the total amount was adjusted.

Step 3) Homogenization was performed (first step: 10 MPa, second step: 5 MPa) at 75°C.

Step 4) The resulting product was whipped in a hot-water bath at 60°C with a handheld mixer for 3 minutes.

Step 5) The whipped product was packed in a container.

Step 6) The packed product was cooled and solidified.

Table 1-4

| Foam Stabilizer | Blank | Comparative Example 1-3 | Comparative Example 1-4 | Comparative Example 1-5 | Comparative Example 1-6 | Example 1-2 | Example 1-3 |
|---|---|---|---|---|---|---|---|
| Xanthan Gum (SAN ACE*2 *1) | - | 0.1% | 0.2% | - | - | - | - |
| Fermentation-derived Cellulose Blend (SAN ARTIST *2 PN*1) | - | - | - | 0.1% | 0.2% | - | - |
| Welan Gum (VIS TOP*2 W*1) | - | - | - | - | - | 0.1% | 0.2% |
| Viscosity of Dessert Mix * | 17.1 mPa·s | 225 mPa·s | 374 mPa·s | 528 mPa·s | 840 mPa·s | 368 mPa·s | 940 mPa·s |
| Overrun | 250% | 72% | 58% | 40% | 16% | 72% | 32% |
| Foaming State | Bubbles are completely escape out of the whipped dessert. | A portion of bubble remains, but many bubbles rise to the upper side. | Some bubbles rise to the upper side. | Some bubbles rise to the upper side. | There are fewer bubbles, but bubbles are retained well. | A few bubbles rise to the upper side. | There are fewer bubbles, but bubbles are retained well. |
| Foam Retainability | C | Between C and B | B | B | A | Between B and A | A |

* Viscosity Measurement Condition: B-type rotational viscometer, 60°C, 60 rpm Evaluation Criteria for Foam Retainability A: Bubbles are evenly present in the whipped dessert without moving.

B: Some bubbles rise to the top, but the others stay inside the whipped dessert.

C: Bubbles escape out of the whipped dessert.

Evaluation Results

With the use of welan gum, the whipped dessert was prepared without losing bubbles even after whipping at 60°C. This effect was equivalent to or higher than the effect of the case where fermentation-derived cellulose was used.

Generally, as the viscosity becomes higher, the overrun becomes lower. However, welan gum achieved a high overrun with a relatively high viscosity; the whipped dessert using welan gum exhibited a higher overrun and contained finer bubbles than the whipped dessert using fermentation-derived cellulose.

Although Example 1-2, to which welan gum was added in an amount of 0.1%, had a lower viscosity than the whipped dessert using fermentation-derived cellulose, Example 1-2 exhibited better dispersion of bubbles (Comparative Examples 1-5 and 1-6, to which fermentation-derived cellulose was added, had more bubbles in the upper portion). Bubbles were more likely to escape from the mix to which xanthan gum was added to provide viscosity (Comparative Examples 1-2 and 1-3), than the mix using welan gum or fermentation-derived cellulose.

Test Example 1-3: Fruit-Juice Beverage

In accordance with the formula shown in the following Table 1-5, fruit-juice beverages were prepared. Table 1-6 shows the type and amount of each foam stabilizer used. Table 1-6 also shows the viscosity, the evaluation results of shaking test, and the state after storage of the obtained fruit-juice beverages.

Table 1-5

| | |
|---|---|
| Fruit Juice (Ehime Beverage Inc., Citrus Mixed Fruit Juice 53R) | 4.4 |
| High-Fructose Corn Syrup | 5.5 |
| Sugar | 3.0 |
| Foam Stabilizer | See Table 1-6 |
| L-ascorbic acid | 0.03 |
| Citric Acid (Anhydrous) | 0.06 |
| Flavor (ORANGE FLAVOR No. 2410*1) | 0.10 |
| Emulsifier (Maruzen Pharmaceuticals Co. Ltd., Quillajanin C-100) | 0.05 |
| With Addition of Ion-Exchanged Water | 100.0% |

Preparation Method

Step 1) A powdery mixture of sugar and a foam stabilizer was added to a container filled with high-fructose corn syrup and ion-exchanged water, and stirred for dissolution at 80°C for 10 minutes, followed by cooling down to room temperature.

Step 2) L-ascorbic acid, fruit juice, citric acid, the flavor, and the emulsifier were added to the preparation obtained in step 1) in this order, and the total amount was adjusted.

Step 3) After the temperature was raised to 75°C, homogenization was performed (first step: 10 MPa, second step: 5 MPa). When the temperature reached 93°C, the resulting product was hot-packed in a PET container.

Table 1-6

| | Foam Stabilizer | Amount | Viscosity* (mPa·s) | Shaking Test** | |
|---|---|---|---|---|---|
| | | | | Immediately After Shaking | After Standing for 10 Minutes |
| Blank | - | - | 6.0 | A (88 ml/-) | C (80 ml/57 ml) |

(continued)

| | Foam Stabilizer | Amount | Viscosity* (mPa·s) | Shaking Test** | |
|---|---|---|---|---|---|
| | | | | Immediately After Shaking | After Standing for 10 Minutes |
| Comparative Example 1-7 | HM Pectin (SM-666*1) | 0.2% | 7.5 | A (88 ml/-) | C (85 ml/57 ml) |
| Comparative Example 1-8 | Fermentation-derived Cellulose Blend (SAN ARTIST*2 PG*1) | 0.2% | 8.0 | A (90 ml/-) | C (88 ml/57 ml) |
| Comparative Example 1-9 | Xanthan Gum (SAN-ACE*2 *1) | 0.2% | 148.0 | A (70 ml/-) | B (65 ml/58 ml) |
| Example 1-4 | Welan Gum (VIS TOP*2 W*1) | 0.2% | 159.5 | A (75 ml/-) | A (75 ml/-) |
| *Viscosity Measurement Condition: B-type rotational viscometer, 5°C, 60 rpm **Shaking Test: The values in the parentheses indicate "the entire volume/the liquid layer portion volume." | | | | | |

Test Method

Step 1) 60 ml of the sample was placed in a 100-ml graduated cylinder so as not to whip.

Step 2) The cylinder was shaken up and down 30 times; and the state of the sample was observed immediately after shaking, and also observed after the sample was allowed to stand for 10 minutes.

Evaluation Method

A: There is no interface.

B: There is a interface, but it is pale and obscure.

C: There is a clear-cut interface.

Evaluation

Welan gum, high-methoxyl (HM) pectin, fermentation-derived cellulose, or xanthan gum wer added to fruit-juice beverages respectively, and their formability after shaking was evaluated. Comparative Examples 1-7 to 1-9, to which HM pectin, fermentation-derived cellulose, or xanthan gum was added, exhibited separation of the foam and the liquid layer after the beverages were allowed to stand for 10 minutes. However, Example 1-4, in which welan gum was used, exhibited the functionality when added in an amount equivalent to that of HM pectin, fermentation-derived cellulose, or xanthan gum; and a liquid layer did not form, with the bubbles stably maintained, even after the beverage was allowed to stand for 10 minutes.

Test Example 1-4: Effervescent Powdered Drink Mix

In accordance with the formula shown in the following Table 1-7, effervescent powdered drink mixes that develop carbon dioxide to form foam were prepared.

Table 1-7

| Sugars | 5.0 |
|---|---|
| Powdered Creamer (AGF, M-40R) | 7.0 |

(continued)

| | |
|---|---|
| Thickener (SAN SUPPORT*2 SX-3*1) | 4.0 |
| Welan Gum (VIS TOP*2 W*1) | 0.5 |
| Foaming Agent (GB MIX NO.26173*1) | 2.3 |
| Thickener (CARRAGEENAN Hi-pHive*1) | 0.25 |
| Citric Acid (CCA*1) | 0.1 |
| Color Additive (CAROTENE POWDER 15-S*1) | 0.1 |
| Flavor (SAN FIX*2 YOGURT No. 21226*1) | 0.4 |
| Flavor (SAN FIX*2 MANGO No. 2001F*1) | 0.3 |
| Sweetener (SAN SWEET*2 SA-8020*1) | 0.05 |
| Sweetener (SAN SWEET*2 SU-100*1) | 0.006 |
| Sweetener (SWEET UP*2 V-30*1) | 0.01 |
| Total | 20.016 parts by mass |

Preparation Method

A mixture of the powders described above was prepared. Subsequently, the mixture was added to 100 g of water at room temperature, and stirred manually for 30 to 60 seconds, thereby obtaining an effervescent beverage.

Evaluation

When the powders were added to water, bubbles immediately started to develop, and the beverage became entirely foamy in about 40 seconds. After stirring for 60 seconds, the entire beverage was foamy like a smoothie, and appeared as though it could be eaten with a straw or spoon. When eaten, the beverage exhibited a refreshing sourness and flavor. When the beverage was allowed to stand for one hour, syneresis, dissipation of bubbles, or the like did not occur, and the beverage was confirmed to be stable.

Test Example 1-5: Facial Cleanser

In accordance with the formula shown in the following Table 1-8, facial cleansers were prepared.

Table 1-8

| | Comparative Example 1-10 | Comparative Example 1-11 | Example 1-5 | Example 1-6 |
|---|---|---|---|---|
| Sodium Cocoyl Glycinate (30%) | 18.0 | 18.0 | 18.0 | 18.0 |
| Sodium Lauroamphoacetate (30%) | 10.0 | 10.0 | 10.0 | 10.0 |
| Polyquaternium-39 | 0.1 | 0.1 | 0.1 | 0.1 |
| 1,3-Butylene Glycol | 5.0 | 5.0 | 5.0 | 5.0 |
| Glycerin | 5.0 | 5.0 | 5.0 | 5.0 |
| Methylparaben | 0.1 | 0.1 | 0.1 | 0.1 |
| Xanthan Gum (SAN ACE*2 *1) | - | 0.05 | - | - |
| Welan Gum (VIS TOP*2 W*1) | - | - | 0.05 | 0.1 |
| With Addition of Ion-Exchanged Water | 100.0% | 100.0% | 100.0% | 100.0% |

Preparation Method

Step 1) Sodium cocoyl glycinate, sodium lauroamphoacetate, polyquaternium-39, 1,3-butylene glycol, glycerin, and methylparaben were placed in a container, and stirred at 60°C for 10 minutes to dissolve the mixture.

Step 2) Xanthan gum or welan gum was added to ion-exchanged water, and stirred for dissolving.

Step 3) While the preparation obtained in step 2) was stirred, the preparation obtained in step 1) was gradually added thereto.

Step 4) 2.5 g of foam were ejected onto a filter paper with a foam pump.

Evaluation Method

The state of foam was visually observed for 10 minutes. Sensory evaluation was also performed for the texture of the foam. Further, after 10 minutes, the moisture that seeped through the filter paper was visually observed to evaluate syneresis. Table 1-9 shows the results.

Table 1-9

|  | State of foam | Texture | Syneresis |
|---|---|---|---|
| Comparative Example 1-10 | Immediately after foaming, foam gradually shrank. | Dry and not smooth | Immediately after foaming, syneresis occurred. |
| Comparative Example 1-11 | After 5 minutes from foaming, foam gradually shrank. | Slightly smooth | After 7 minutes from foaming, syneresis occurred. |
| Example 1-5 | Even after 10 minutes from foaming, foam did not shrink. | Smooth, with a dense, creamy and full-bodied texture | Even after 10 minutes from foaming, syneresis did not occur. |
| Example 1-6 | Even after 10 minutes from foaming, foam did not shrink. | Smooth, with a dense, creamy and full-bodied texture | Even after 10 minutes from foaming, syneresis did not occur. |

Evaluation

In Comparative Example 1-10, in which a polysaccharide thickener was not added, foam started to shrink immediately after foam formation. The texture of the foam was dry, and not smooth. Additionally, syneresis occurred immediately after foam formation.
In Comparative Example 1-11, in which xanthan gum was added, foam started to shrink after 5 minutes from foam formation, and the foam stability was poor. The texture of the foam was slightly smooth. Syneresis occurred after 7 minutes from foam formation.
In Examples 1-5 and 1-6, to which welan gum was added, the facial cleansers had high stability without shrinkage of foam even after 10 minutes from foaming. The cleansers also had a smooth texture and a dense, creamy and full-bodied texture. Even after 10 minutes from foaming, syneresis did not occur.

Test Example 1-6: Spray Detergent

In accordance with the formulas shown in the following Table 1-10, spray detergents were prepared.

Table 1-10

|  | Comparative Example 1-12 | Example 1-7 |
|---|---|---|
| Ethylenediaminetetraacetic Acid | 18.0 | 18.0 |
| Citric Acid | 10.0 | 10.0 |

(continued)

|  | Comparative Example 1-12 | Example 1-7 |
|---|---|---|
| Sodium Alkylbenzene Sulfonate | 0.1 | 0.1 |
| Dodecyl Polyoxyethylene Ether | 5.0 | 5.0 |
| Ethyl Carbitol | 5.0 | 5.0 |
| Welan Gum (VIS TOP*2 W*1) | - | 0.2 |
| Sodium Hydroxide | Suitable Amount | Suitable Amount |
| With Addition of Ion-Exchanged Water | 100.0% | 100.0% |

Preparation Method

Step 1) Welan gum was added to ion-exchanged water, and stirred for dissolution.

Step 2) Ethylenediaminetetraacetic acid and citric acid were dissolved in ion-exchanged water, and pH was adjusted to 7 with sodium hydroxide.

Step 3) The preparation obtained in step 2), sodium alkylbenzene sulfonate, dodecyl polyoxyethylene ether, and ethyl carbitol were added to the preparation obtained in step 1), and mixed with stirring.

Step 4) The preparation obtained in step 3) was packed in a trigger-spray container.

Evaluation Method

The detergent was sprayed toward a vertically placed acrylic panel from 15 cm away. On this occasion, the detergent was sprayed such that foam is formed at the point of 22 cm in height of the acrylic panel, and the time period it takes until foam drop off to the bottom edge of the acrylic panel (0 cm in height) was determined to be a retention time.

Evaluation Results

In Comparative Example 1-12, to which welan gum was not added, the retention time was 15 seconds. However, in Example 1-7, to which welan gum was added, the retention time was 32 seconds, and the drop-off of the foam was noticeably suppressed.

Test Example 2-1: Baked Meringue

In accordance with the formula shown in the following Table 2-1, baked meringue was prepared. Table 2-2 shows the type and amount of each texture improver used. The whipped meringue was evaluated with respect to foamability, syneresis, and texture after baking; Table 2-2 also shows the results. The evaluation of syneresis was performed by packing whipped meringue in a pudding cup, allowing it to stand at 20°C for 2 hours, and visually observing it. Regarding the texture after baking, a greater number of symbols "+" indicates a higher mark.

Table 2-1

| Egg White | 60.0 (g) |
|---|---|
| Sugar | 40.0 |
| Salt | 0.1 |
| Texture Improver | See Table 2-1 |
| Ion-Exchanged Water | 20.0 |

Preparation Method

Step 1) A texture improver was added to ion-exchanged water, and the mixture was stirred at 80°C for 10 minutes, followed by cooling down to 10°C.
Step 2) Egg white was cooled to 10°C.
Step 3) Egg white, salt, and the preparation obtained in step 1) were placed in a bowl.
Step 4) The mixture was whipped with a handheld mixer for 1 minute.
Step 5) Sugar was added thereto over 30 seconds, and the mixture was further whipped for 1 minute.

Baking Method

Step 1) The whipped meringue was packed in a bag, and squeezed out to form a flower (or star) shape (3 g each) with the piping bag.
Step 2) The meringue was baked in an oven at 100°C.

Table 2-2

| | Texture Improver | Amount (of the weight of batter before baking) | After Whipping | Texture After Baking | | |
| --- | --- | --- | --- | --- | --- | --- |
| | | | Syneresis (after being allowed to stand at 20°C for 2 hours) | Crunchiness | Perceived Meltability in Mouth | Uneven Baking |
| Blank | - | - | Occurred in the bottom | ++ | ++ | ++ |
| Comparative Example 2-1 | Xanthan Gum (SAN ACE*2 *1) | 0.1 g (0.08%) | Did not occur | +++ | ++ | +++ |
| Comparative Example 2-2 | Xanthan Gum (SAN ACE*2 *1) | 0.35 g (0.25%) | Did not occur | +++ | + | ++ |
| Comparative Example 2-3 | Soybean Soluble Polysaccharide (SM-700*1) | 0.1 g (0.08%) | Occurred | +++ | ++ | + |
| Comparative Example 2-4 | Soybean Soluble Polysaccharide (SM-700*1) | 1.0 g (0.83%) | Occurred | ++++ | +++ | + |
| Comparative Example 2-5 | Microcrystalline Cellulose Blend (Asahi Kasei Corporation, CEOLUS RC-N30) | 0.1 g (0.08%) | Occurred | ++++ | + | + |
| Comparative Example 2-6 | Microcrystalline Cellulose Blend (Asahi Kasei Corporation, CEOLUS RC-N30) | 1.0 g (0.83%) | Occurred | ++++ | + | + |
| Example 2-1 | Welan Gum (VIS TOP*2 W*1) | 0.1 g (0.08%) | Did not occur | ++++ | ++++ | ++++ |
| Example 2-2 | Welan Gum (VIS TOP*2 W*1) | 0.025 g (0.02%) | Occurred | +++ | +++ | +++ |

(continued)

| | Texture Improver | Amount (of the weight of batter before baking) | After Whipping | Texture After Baking | | |
| | | | Syneresis (after being allowed to stand at 20°C for 2 hours) | Crunchiness | Perceived Meltability in Mouth | Uneven Baking |
| --- | --- | --- | --- | --- | --- | --- |
| Example 2-3 | Welan Gum (VIS TOP*2 W*1) | 0.05 g (0.04%) | Did not occur | +++ | +++ | ++++ |
| Example 2-4 | Welan Gum (VIS TOP*2 W*1) | 0.2 g (0.17%) | Did not occur | ++++ | ++++ | ++++ |
| Example 2-5 | Welan Gum (VIS TOP*2 W*1) | 0.3 g (0.25%) | Did not occur | +++ | +++ | ++++ |
| Example 2-6 | Welan Gum (VIS TOP*2 W*1) | 0.1 g (0.08%) | Did not occur | ++++ | ++++ | ++++ |
| | Soybean Soluble Polysaccharide (SM-700*1) | 0.1 g (0.08%) | | | | |
| Example 2-7 | Welan Gum (VIS TOP*2 W*1) | 0.1 g (0.08%) | Did not occur | ++++ | ++++ | ++++ |
| | Microcrystalline Cellulose Blend (Asahi Kasei Corporation, CEOLUS RC-N30) | 0.1 g (0.08%) | | | | |

Evaluation Results

In Comparative Example 2-1, in which xanthan gum was used, the texture after baking, in particular, the perceived meltability in the mouth, was better than the blank; however, the difference was minimal, and the effect was insufficient. Comparative Example 2-2, in which an increased amount of xanthan gum was used, exhibited improved crunchiness, but also exhibited strong stickiness unique to xanthan gum, with the perceived meltability in the mouth of baked meringue significantly lost.

Comparative Examples 2-3 and 2-4, in which a soybean soluble polysaccharide was used, exhibited improved crunchiness compared with the blank; however, the bubbles of the meringue became larger during baking, resulting in uneven baked meringue with a gritty texture.

Comparative Examples 2-5 and 2-6, in which a microcrystalline cellulose blend was used, exhibited poorer stability during baking than the cases where a soybean soluble polysaccharide was used, and the meringue was not evenly baked and had a gritty texture, with the syneresis in the bottom part burned like caramel.

In Examples 2-1 to 2-5, in which welan gum was used, meringue after baking exhibited better collapsibility than in Comparative Example 2-1, in which xanthan gum was added. Moreover, the texture of small fine bubbles of the meringue after baking was maintained; compared with the blank, the obtained baked meringue had a clearly better perceived meltability in the mouth and crunchy texture.

Example 2-6, in which welan gum and a soybean soluble polysaccharide were used in combination, and Example 2-7, in which welan gum and microcrystalline cellulose were used in combination, exhibited, due to the addition of welan gum, a decrease in uneven baking, which had been the problem for each case; and the obtained baked meringue had preferable crunchiness and perceived meltability in the mouth.

Test Example 2-2: Macaroon

In accordance with the formula shown in the following Table 2-3, macaroons were prepared. Table 2-4 shows the type and amount of each texture improver used. The texture of macaroon after baking was evaluated; Table 2-4 also shows the results.

Table 2-3

| Egg White | 100.0 (g) |
|---|---|
| Almond Flour | 100.0 |
| Powder Sugar | 160.0 |
| Sugar | 50.0 |
| Texture Improver | See Table 2-2 |

Preparation Method

Step 1) While stirring egg white cooled to 5°C with a handheld mixer, sugar was gradually added thereto, and the mixture was beat until peaks formed, thereby preparing meringue. Step 2) A sieved mixture of almond flour and powder sugar was added to the preparation obtained in step 1), and the mixture was well mixed with a rubber spatula until the entire mixture blended well. Then, the batter underwent the macaronage step until it became shiny.

Step 3) A baking paper was laid on a sheet pan, and the batter was squeezed out into a diameter of about 3 cm on the paper, and allowed to stand at room temperature for about 30 minutes to dry the surface of the batter.

Step 4) After the batter was baked in an oven at 210°C for 2 minutes, the oven was cooled down to the temperature of 150°C, and the batter was further baked for 13 minutes.

Table 2-4

| | Texture Improver | Amount (of the weight of batter before baking) | After Baking |
|---|---|---|---|
| | | | Texture |
| Blank | - | - | Benchmark |
| Comparative Example 2-7 | Xanthan Gum (SAN ACE*2 *1) | 0.2 g (0.05%) | After chewing, stickiness was felt, with poor perceived meltality in the mouth. |
| Example 2-8 | Welan Gum (VIS TOP*2 W*1) | 0.2 g (0.05%) | Much crunchier, and better perceived meltality in the mouth than the blank. |

Evaluation

In Comparative Example 2-7, in which xanthan gum was used, the macaroon was sticky, felt like it was adhering to the teeth when chewed, and the perceived meltability in the mouth was not particularly good, when compared with the blank. However, in Example 2-8, to which welan gum was added, the macaroon had a lighter and crunchier texture than the blank, with no stickiness. The perceived meltability in the mouth was also excellent.

Test Example 3-1: Lacto-ice

In accordance with the formula shown in the following Table 3-1, lacto-ice was prepared. Table 3-2 shows the type and amount of each stabilizer used, and the results of sensory evaluation. The prepared lacto-ice had a total solids content of 33.6%, a nonfat milk solids content of 8.6%, a vegetable fat content of 9.0%, and a degree of relative sweetness of 14.3.

Table 3-1

| Sugar | 13.5 |
|---|---|
| Skimmed Milk Powder | 9.0 |
| Coconut Oil | 9.0 |
| Starch Syrup | 3.0 |

(continued)

| Stabilizer | See Table 3-1 |
|---|---|
| Emulsifier (HOMOGEN*2 DM-S*1) | 0.2 |
| With addition of Ion-Exchanged Water | 100.0% |

Preparation Method

Step 1) While starch syrup and water were stirred, a powder mixture of sugar, skimmed milk powder, a stabilizer, and the emulsifier prepared beforehand was added thereto, and the mixture was heated with stirring.

Step 2) After the temperature reached 80°C, coconut oil was added, and the mixture was stirred to dissolve the components for 10 minutes with the temperature maintained.

Step 3) After the total amount was adjusted with water, homogenization was performed (first step: 10 MPa, second step: 5 MPa) .

Step 4) After having been cooled down to 5°C or below, the homogenized mix was aged overnight.

Step 5) Freezing (overrun: about 80%) was performed, and the resulting product was packed in a cup and rapidly frozen at -40°C.

Table 3-2

| NO. | Stabilizer | Amount | Sensory Evaluation Results |
|---|---|---|---|
| Blank | - | - | The ice crystals were very coarse, and the lacto-ice had a gritty texture. |
| Example 3-1 | Welan Gum | 0.05% | Compared with the blank, the lacto-ice had a decreased ice crystalline texture, and had a full-bodied texture. |
| Example 3-2 | | 0.10% | The lacto-ice had a stronger bodying sensation than Example 3-1, exhibiting excellent perceived meltability in the mouth. |
| Example 3-3 | | 0.25% | The lacto-ice was smoother and had a better bodying sensation than Example 3-2, exhibiting excellent perceived meltability in the mouth. |
| Example 3-4 | | 0.50% | The lacto-ice had a richer texture than Example 3-3, exhibiting excellent mouth-melt. |
| Comparative Example 3-1 | Xanthan Gum | 0.25% | The lacto-ice lingered in the mouth, with very poor perceived meltability in the mouth and flavor release. |
| Comparative Example 3-2 | Guar Gum | 0.25% | The lacto-ice had a lighter texture than Example 3-3 at chewing, but the perceived meltability in the mouth was poor. |
| Comparative Example 3-3 | Locust Bean Gum | 0.25% | The lacto-ice had less viscosity than Example 3-3, with weak bodying sensation. |

(continued)

| NO. | Stabilizer | Amount | Sensory Evaluation Results |
|---|---|---|---|
| Comparative Example 3-4 | Tamarind Seed Gum | 0.15% | The lacto-ice exhibited poor flavor release, and had a slightly dry texture. |
| | Locust Bean Gum | 0.10% | |

| Welan Gum: VIS TOP *2 W*1<br>Xanthan Gum: SAN ACE*2 *1<br>Guar Gum: VIS TOP*2 D-20*1<br>Locust Bean Gum: VIS TOP*2 D-30*1<br>Tamarind Seed Gum: VIS TOP*2 D-2032*1 |
|---|

Evaluation

As shown in Table 3-2, adding welan gum to lacto-ice imparts smoothness and body to the lacto-ice. Compared with conventionally used polysaccharides, welan gum can prepare lacto-ice that has excellent perceived meltability in the mouth and a much cleaner, pleasant sharpness.

Test Example 3-2: Syneresis Suppression Test on Lacto-Ice

In the production of lacto-ice, a comparative test was performed using polysaccharides that are typically used in expectation of a syneresis suppression effect. In accordance with the formula of lacto-ice shown in the following Table 3-3, a stabilizer was added as indicated in Table 3-4, and lacto-ice was prepared in the same manner as the preparation method in Test Example 3-1.

Table 3-4 shows the type and amount of each stabilizer used, the viscosity of the mixes, and the results of syneresis suppression evaluation.

Table 3-3

| | |
|---|---|
| Sugar | 13.5 |
| Skimmed Milk Powder | 9.0 |
| Coconut Oil | 9.0 |
| Starch Syrup | 3.0 |
| Locust Bean Gum (VIS TOP*2 D-30*1) | 0.15 |
| Stabilizer | See Table 3-2 |
| Emulsifier (HOMOGEN*2 DM-S*1) | 0.2 |
| With addition of Ion-Exchanged Water | 100.0% |

Syneresis Suppression Evaluation

The obtained lacto-ice was placed on filter paper at room temperature (25°C), and allowed to stand. After 60 minutes, the mass of the filter paper was measured, and a syneresis rate was calculated using the following equation. Table 3-4 shows the results.

```
A syneresis rate = the amount of water absorbed by the filter
paper/the mass of lacto-ice × 100
```

(the amount of water absorbed by the filter paper = the mass of the filter paper after 60 minutes - the initial mass of the filter paper)

Table 3-4

|  | Stabilizer | Amount | Syneresis Rate (%) | Viscosity of the mixes (mPa·s) | Sensory Evaluation |
|---|---|---|---|---|---|
| Blank | - | - | 11.5 | 99 | The lacto-ice had a grainy texture caused by large ice crystals, with less smoothness and bodying sensation. |
| Example 3-5 | Welan Gum | 0.03% | 6.5 | 217 | The lacto-ice had smoothness and body, with excellent perceived meltability in the mouth. |
| Comparative Example 3-5 | Xanthan Gum | 0.03% | 9.4 | 313 | The lacto-ice had a grainy texture caused by large ice crystals. Bodying sensation and smoothness of the lacto-ice are not so bad, nevertheless the perceived meltability in the mouth was considerably bad. |
| Comparative Example 3-6 | Native Gellan Gum | 0.03% | 8.0 | 376 | The lacto-ice had a bodying sensation, thickness and smoothness, but the perceived meltability in the mouth was poor. |
| Comparative Example 3-7 | Carrageenan | 0.045% | 9.5 | 1294 | The lacto-ice had a bodying sensation and smoothness, but the perceived meltability in the mouth was poor. |
| Viscosity Measurement Conditions: BL-type viscometer, suitably selected rotors, 5°C, 60 rpm Locust Bean Gum: VIS TOP*2 D-30*1<br>Welan Gum: VIS TOP*2 W*1<br>Xanthan Gum: SAN ACE*2 *1<br>Native Gellan Gum: KELCOGEL LT100*1<br>Carrageenan: CARRAGEENAN CSI-1(F)*1 | | | | | |

Evaluation

The results indicate that welan gum has a better syneresis suppression effect than native gellan gum, which has been known to have the most potent syneresis suppression effect on frozen desserts.

Specifically, the lacto-ice of Example 3-5 exhibited the lowest syneresis rate, and the viscosity of the mix was acceptable, which did not make it difficult to handle the lacto-ice when prepared.

The blank, however, exhibited a low viscosity of the mix, with a high syneresis rate, which did not make it difficult to handle the lacto-ice though; the syneresis was not suppressed. Although Comparative Examples 3-5 to 3-7 exhibited a higher viscosity than Example 3-5, their syneresis rate was higher than that of Example 3-5, and the perceived meltability in the mouth was poor.

From the results above, it is inferred that the use of welan gum can prevent a prepared frozen dessert with a covering that absorbs moisture, such as *monaka* ice cream, from becoming moist by water generated by syneresis and degrading in texture.

Polysaccharides, which have a syneresis suppression effect, generally have high viscosity, and frozen desserts to which a high syneresis suppression effect has been imparted by them are likely to have a heavy texture. However, welan gum, despite having a strong syneresis suppression effect, enabled preparation of a frozen dessert with excellent perceived meltability and clean sensation.

Lacto-ice to which locust bean gum, guar gum, or tamarind seed gum was added in an amount of 0.2% and lacto-ice to which welan gum was added in an amount of 0.025 to 0.25% as shown in Table 3-5 were compared in terms of their syneresis rate. Table 3-5 shows the amount of each polysaccharide and syneresis rate.

Table 3-5

| Amount (%) | Blank | Example 3-6 | Example 3-7 | Example 3-8 | Example 3-9 | Example 3-10 | Example 3-11 | Comparative Example 3-8 | Comparative Example 3-9 | Comparative Example 3-10 |
|---|---|---|---|---|---|---|---|---|---|---|
| Welan Gum | - | 0.025 | 0.05 | 0.1 | 0.15 | 0.2 | 0.25 | - | - | - |
| Locust Bean Gum | - | - | - | - | - | - | - | 0.2 | - | - |
| Guar Gum | - | - | - | - | - | - | - | - | 0.2 | - |
| Tamarind Seed Gum | - | - | - | - | - | - | - | - | - | 0.2 |
| Syneresis Rate | 17.3 | 11.0 | 8.6 | 8.0 | 5.8 | 5.0 | 4.3 | 11.5 | 12.9 | 10.4 |
| Welan Gum: VIS TOP*2 W*1<br>Locust Bean Gum: VIS TOP*2 D-30*1<br>Guar Gum: VIS TOP*2 D-20*1<br>Tamarind Seed Gum: VIS TOP*2 D-2032*1 | | | | | | | | | | |

Evaluation results

Examples 3-6 to 3-11, to which welan gum was added, exhibited significantly less syneresis than the blank product. Although Comparative Examples 3-8 to 3-10, to which locust bean gum, guar gum, or tamarind seed gum alone was added, exhibited less syneresis than the blank product, the effect was substantially equivalent to that of Example 3-6, in which 0.025% of welan gum was added.

Test Example 3-3: Foamability Test on Lacto-Ice

A comparative test was performed with polysaccharides used in the production of lacto-ice in expectation of improving the foamability.
In accordance with the same formula as in Test Example 3-1, but with the stabilizers shown in Table 3-6, lacto-ice was prepared in the same manner as the preparation method in Test Example 3-1. Table 3-6 shows the type and amount of each stabilizer used and the results of appearance evaluation, which was performed for evaluating the shape formability.

Preparation Method

Lacto-ice was prepared in accordance with the same procedure and operation as in Test Example 3-1.

Experiment Operation

Lacto-ice that had been subjected to freezing was packed in a piping bag, and squeezed out in a cone shape on a filter paper. 1 minute, 3 minutes, and 5 minutes after the lacto-ice was squeezed out, the change in shape was visually observed. Table 3-6 shows the results.

Table 3-6

| Amount (%) | Blank | Example 3-12 | Example 3-13 | Example 3-14 | Example 3-15 | Example 3-16 | Example 3-17 | Comparative Example 3-11 | Comparative Example 3-12 | Comparative Example 3-13 | Comparative Example 3-14 |
|---|---|---|---|---|---|---|---|---|---|---|---|
| Welan Gum | - | 0.025 | 0.05 | 0.1 | 0.15 | 0.2 | 0.25 | - | - | - | - |
| Locust Bean Gum | - | - | - | - | - | - | - | 0.25 | - | - | - |
| Guar Gum | - | - | - | - | - | - | - | - | 0.25 | - | - |
| Tamarind Seed Gum | - | - | - | - | - | - | - | - | - | 0.25 | - |
| Xanthan Gum | - | - | - | - | - | - | - | - | - | - | 0.25 |
| Appearance Evaluation | | | | | | | | | | | |
| After 1 minute | 9 | 10 | 10 | 10 | 10 | 10 | 10 | 9 | 9 | 9 | 10 |
| After 3 minutes | 5 | 9 | 9 | 9 | 9 | 9 | 10 | 4 | 4 | 6 | 6 |
| After 5 minutes | 3 | 7 | 8 | 7 | 8 | 8 | 9 | 2 | 2 | 4 | 4 |

Appearance Evaluation --- the edge sharpness, surface smoothness, etc., of the squeezed-out lacto-ice were visually observed, and rated on a scale of 1 to 10 with 10 being the best and 1 being the worst (the worst is the state of the blank at the 10-minute point after it was squeezed out).
Welan Gum: VIS TOP*2 W*1
Locust Bean Gum: VIS TOP*2 D-30*1
Guar Gum: VIS TOP*2 D-20*1
Tamarind Seed Gum: VIS TOP*2 D-2032*1
Xanthan Gum: SAN ACE*2 *1

Evaluation

Lacto-ice to which welan gum was added had a stiff peak with sharp edges immediately after being squeezed out, and was excellent in shape formability. Additionally, since the shape was maintained even for 5 minutes after the lacto-ice was squeezed out, the lacto-ice was able to proceed to the subsequent production step without losing its shape after being squeezed into a cone or *monaka* waffle; thus, the lacto-ice was confirmed to have a preferable effect from a production perspective. Comparative Examples 3-11 to 3-14, in which other polysaccharides were used, were slightly inferior in the shape immediately after the lacto-ice was squeezed out, even with a larger amount of polysaccharides than welan gum. Moreover, after 3 minutes, the lacto-ice lost the edges of the peak and the cone shape, failing to maintain the preferable shape.

Test Example 3-4: Ice Pop

In accordance with the formulas shown in the following Tables 3-7 and 3-9, soda-flavored ice pops and milk-flavored ice pops were prepared. The ice pops were evaluated for the suppression effect on sugar leak. Table 3-8 shows the type and amount of the stabilizer added, and the amount of sugar leak for the soda-flavored ice pop. Table 3-10 shows the type and amount of the stabilizer added, and the results of texture evaluation for the milk-flavored ice pop.

Table 3-7

| | |
|---|---|
| High-Fructose Corn Syrup | 11.0 |
| Sugar | 8.0 |
| Color Additive (Food Blue No.1) | 0.0001 |
| Citric Acid (Anhydrous) | 0.1 |
| Stabilizer | See Table 3-8 |
| Flavor (CHAMPAGNE CIDER EXTRACT*1) | 0.1 |
| With addition of Ion-Exchanged Water | 100.0% |

Preparation Method

Step 1) While high-fructose corn syrup and water were stirred, a mixture of sugar and a stabilizer prepared beforehand was added thereto, and the resulting mixture was stirred for dissolution at 80°C for 10 minutes, followed by cooling down to 5°C or below.

Step 2) Citric acid, the color additive, and the flavor were added to the preparation obtained in step 1), and the total amount was adjusted with water.

Step 3) The preparation obtained in step 2) was packed in ice molds (80 g each), and hardened at -30°C.

Sugar Leak Evaluation

The prepared sample products were sandwiched by a filter paper, placed in bags, sealed, and stored upright at -15°C for 3 days. After storage, the change in weight of each filter paper was measured, and the change of weight was determined to be the amount of leaked sugar.

Table 3-8

| | Stabilizer | Amount (%) | Amount of Leaked Sugar (g) |
|---|---|---|---|
| Blank | - | - | 3.02 |

(continued)

| | Stabilizer | Amount (%) | Amount of Leaked Sugar (g) |
|---|---|---|---|
| Example 3-18 | Welan Gum | 0.03 | 1.10 |
| Example 3-19 | | 0.05 | 0.80 |
| Example 3-20 | | 0.1 | 0.53 |
| Example 3-21 | | 0.2 | 0.34 |
| Example 3-22 | | 0.3 | 0.30 |
| Comparative Example 3-15 | Carrageenan | 0.03 | 1.85 |
| Comparative Example 3-16 | | 0.05 | 1.07 |
| Comparative Example 3-17 | | 0.1 | 0.34 |
| Comparative Example 3-18 | Tamarind Seed Gum | 0.2 | 0.90 |
| Comparative Example 3-19 | | 0.3 | 0.55 |
| Welan Gum: VIS TOP*2 W*1<br>Carrageenan: CARRAGEENAN CS-613*1<br>Tamarind Seed Gum: VIS TOP*2 D-2032*1 | | | |

Table 3-9

| | |
|---|---|
| Skimmed Milk Powder | 9.0 |
| Sugar | 11.0 |
| Coconut Oil | 8.0 |
| Starch Syrup | 5.0 |
| Emulsifier (HOMOGEN*2 DM-S*1) | 0.2 |
| Stabilizer | See Table 3-10 |
| Flavor (VANILLA FLAVOR No. 93-1*1) | 0.1 |
| With addition of Including Ion-Exchanged Water | 100.0% |

Preparation Method

Step 1) While coconut oil, starch syrup and water were stirred, a mixture of sugar, skimmed milk powder, the emulsifier, and a stabilizer prepared beforehand was added thereto, and the mixture was stirred for dissolution at 80°C for 10 minutes.

Step 2) After the total amount was adjusted, the mixture was homogenized (first homogenization at 10 MPa, second homogenization at 5 MPa), followed by cooling down to 5°C.

Step 3) The flavor was added to the preparation obtained in step 2), and the resulting product was packed in ice molds, 80 g each, and hardened at -30°C.

Table 3-10

| | Stabilizer | Amount (%) | Evaluation |
|---|---|---|---|
| Blank | - | - | Crisp and hard at first bite. |

(continued)

| | Stabilizer | Amount (%) | Evaluation |
|---|---|---|---|
| Example 3-23 | Welan Gum | 0.05 | Smoother and richer than the blank. |
| Example 3-24 | | 0.1 | Richer than the blank, moderately smoother than Example 3-23, with no unpleasant slimy texture. |
| Example 3-25 | | 0.2 | Less crisp than the blank and Examples 3-23 and 3-24, having a soft and smooth texture at first bite, with no unpleasant slimy texture. |
| Example 3-26 | | 0.3 | Less crisp than the blank and Examples 3-23 to 3-25, having a softer and smoother texture than Example 3-25 with no unpleasant slimy texture. Excellent in flavor compared with Comparative Examples 3-20 and 3-21. |
| Comparative Example 3-20 | Tamarind Seed Gum | 0.2 | Having a slightly hard texture, crisp, with an odor derived from the polysaccharide. |
| Comparative Example 3-21 | Tamarind Seed Gum / Locust Bean Gum | 0.2 / 0.1 | Having a soft texture, but having an odor derived from the polysaccharide. |
| Welan Gum: VIS TOP*2 W*1 Locust Bean Gum: VIS TOP*2 D-30*1 Tamarind Seed Gum: VIS TOP*2 D-2032*1 | | | |

Evaluation

In the soda-flavored ice pops of Table 3-7, the addition of welan gum exhibited an effect equivalent to or higher than the effect of the conventionally used carrageenan, which has a profound suppression effect on sugar leak in frozen desserts. In particular, even a small amount of welan gum (Examples 3-18 and 3-19) exhibited a great effect, suggesting that welan gum is more effective than carrageenan. Moreover, while carrageenan resulted in a slightly hard texture, welan gum gave a light crunchy texture, making the ice pop easy to bite. Generally, when a stabilizer is added in an amount of about 0.3%, a flavor derived from the stabilizer is perceived, deteriorating the flavor release. However, when welan gum in an amount of about 0.3% was added (Example 3-22), the prepared ice pop had a cooling sensation and excellent flavor, with ignorable flavor of welan gum.

Polysaccharides generally reduce the crispness when an ice pop is bitten and give a soft texture, but also impart high viscosity; the use of less viscous polysaccharides may result in a crisp or hard texture. Regarding the milk-flavored ice pops of Table 3-9, the prepared milk-flavored ice pops to which welan gum was added (Examples 3-23 to 3-26), however, had excellent perceived meltability in the mouth, with a sharp aftertaste. When chewed, the ice pops were soft, and also had an excellent flavor with smoothness and richness.

Test Example 3-5: Sherbet Bar

In accordance with the formula shown in the following Table 3-11, sherbet bars were prepared, and the sugar leak suppression effect and the texture were evaluated. Table 3-12 shows the type and amount of each stabilizer added, and the results of texture evaluation.

Table 3-11

| High-Fructose Corn Syrup | 13.0 |
|---|---|
| Starch Syrup | 8.0 |
| Sugar | 3.0 |

(continued)

| | |
|---|---|
| 5-fold Concentrated Apple Fruit Juice (Clarified) | 4.0 |
| Citric Acid (Anhydrous) | 0.2 |
| Stabilizer | See Table 3-12 |
| Flavor (APPLE FLAVOR No. 72118*1) | 0.1 |
| Color Additive (SAN YELLOW*2 No. 2AU*1) | 0.015 |
| With addition of Ion-Exchanged Water | 100.0% |

Preparation Method

Step 1) While high-fructose corn syrup, starch syrup, and ion-exchanged water were stirred, a mixture of sugar and a stabilizer prepared beforehand was added thereto, and the mixture was stirred for dissolution at 80°C for 10 minutes, followed by cooling down to 5°C or below.

Step 2) Fruit juice, citric acid, the color additive, and the flavor were added to the preparation obtained in step 1), and the total amount was adjusted with water.

Step 3) Freezing (overrun: about 8%) was performed, and the resulting product was packed in ice molds, and hardened at -30°C.

Sugar Leak Evaluation

The prepared sample products were sandwiched by a filter paper, placed in bags, sealed, and stored upright at -15°C for 4 days. Sugar leak was evaluated based on the change in weight of each filter paper after storage.

Table 3-12

| NO. | Stabilizer | Amount | Amount of Sugar Leak (g) | Evaluation | |
|---|---|---|---|---|---|
| | | | | Pleasant Icy Feeling | Flavor |
| Blank | - | - | 4.90 | 10 | 10 |
| Example 3-27 | Welan Gum | 0.05% | 0.73 | 10 | 10 |
| Example 3-28 | | 0.1% | 0.41 | 9 | 9 |
| Example 3-29 | | 0.2% | 0.36 | 7 | 8 |
| Comparative Example 3-22 | Carrageenan (1) | 0.1% | 0.50 | 6 | 7 |
| Comparative Example 3-23 | Carrageenan (2) | 0.1% | 0.43 | 3 | 6 |
| Comparative Example 3-24 | Tamarind Seed Gum | 0.1% | 0.71 | 4 | 4 |
| Welan Gum: VIS TOP*2 W*1 Carrageenan (1): CARRAGEENAN CS-613*1 Carrageenan (2): CARRAGEENAN CSI-1(F)*1 Tamarind Seed Gum: VIS TOP*2 D-2032*1 | | | | | |

Evaluation Criteria

Evaluation was performed on a scale of 1 to 10 (11-grade evaluation including 0). The strongest grainy and icy texture is rated 10 and none of such a texture is 0; regarding the flavor, excellent flavor release is rated 10, and a strong stabilizer-derived odor with no flavor release is rated 0.

Evaluation

The addition of welan gum significantly reduced sugar leak caused by heat shock, and improved the heat-shock resistance. The prepared sherbets had almost no slimy texture or stringiness, unique to polysaccharides, but had a pleasant icy texture (Examples 3-27 to 3-29). Although the use of other polysaccharides somewhat exhibited a suppression effect on sugar leak, the pleasant icy texture was lost, with the flavor release also deteriorated (Comparative Examples 3-22 to 3-24).

It has been an issue that during the time from immediately after freezing to hardening, the separation of the sherbet mix occurs (while frozen ice and foam float to form an upper layer, the liquid portion of the mix, due to a large specific gravity, forms a lower layer). However, in Examples 3-27 to 3-29, to which welan gum was added, the separation did not occur, and visual observation confirmed homogeneous sherbet bars.

Test Example 3-6: Shaved Ice

In accordance with the formulas shown in the following Tables 3-13 and 3-15, soda-flavored and milk-flavored shaved ice was prepared. Tables 3-14 and 3-16 show the type and amount of each stabilizer used, viscosity of syrup, overrun, ice dispersibility (only for soda-flavored), and the results of scoopability evaluation.

Table 3-13

| Sugar | 22.0 |
|---|---|
| Starch Syrup | 12.0 |
| High-Fructose Corn Syrup | 10.0 |
| Color Additive (Food Blue No.1) | 0.0002 |
| Citric Acid (Anhydrous) | 0.2 |
| Stabilizer | See Table 3-14 |
| Flavor (CHAMPAGNE CIDER EXTRACT*1) | 0.2 |
| With addition of Ion-Exchanged Water | 100.0% |

Preparation Method

Step 1) While starch syrup, high-fructose corn syrup, and water were stirred, a mixture of sugar and a stabilizer prepared beforehand was added thereto, and the mixture was stirred for dissolution at 80°C for 10 minutes, followed by cooling down to 5°C.

Step 2) Citric acid, the color additive, and the flavor were added to the preparation obtained in step 1), and the total amount was adjusted.

Step 3) Ice was shaved with an ice shaver, and the shaved ice and the syrup (the preparation obtained in step 2)) were mixed with a handheld mixer for 30 seconds such that the ratio of ice to syrup was 1/1. The resulting product was packed in a container, and hardened at -40°C.

Ice Dispersibility Evaluation

100 ml of the mixture solution of ice and syrup (the preparation in step 3) before hardening) was filled into a 100-ml graduated cylinder, and the dispersed state of ice was observed. The ice dispersibility refers to a boundary line between the syrup and the ice (a distance from the bottom of the graduated cylinder: mm) recorded at the 5-minute point after the resulting product was filled into the graduated cylinder. A higher value of ice dispersibility means that the mixture is easy to separate after mixing of syrup and ice.

The scoopability was evaluated based on the hand feeling in inserting a spoon into the prepared shaved ice.

Table 3-14

| NO. | Stabilizer | Amount (%) | Viscosity (mPa·s) | Overrun (%) | Ice Dispersibility (mm) | Scoopability |
|---|---|---|---|---|---|---|
| Blank | - | - | 9.6 | 5.7 | 10 | Very hard and difficult to scoop |
| Example 3-30 | Welan Gum | 0.05 | 51 | 10.6 | 8 | Scoopability was better than in the blank, and equivalent to that in Comparative Example 3-25 |
| Example 3-31 | | 0.1 | 106 | 12.3 | 8 | Scoopability was better than in Comparative Example 3-25 |
| Example 3-32 | | 0.2 | 276 | 19.5 | 2 | Scoopability was better than in Example 3-31 |
| Example 3-33 | | 0.3 | 467 | 22.2 | 0 | Scoopability was better than in Example 3-32 |
| Example 3-34 | | 0.5 | 1052 | 25.5 | 0 | Scoopability was better than in Example 3-33 |
| Comparative Example 3-25 | Locust Bean Gum Carrageenan | 0.1 0.1 | 628 | 11.2 | 3 | Scoopability was better than in the blank, and equivalent to that in Example 3-30 |
| Viscosity Measurement Condition: B-type rotational viscometer, 5°C, 60 rpm Welan Gum: VIS TOP*2 W*1 Locust Bean Gum: VIS TOP*2 D-30*1 Carrageenan: CARRAGEENAN CSI-1(F)*1 | | | | | | |

Evaluation Results

In the case of the formula of soda-flavored shaved ice in Table 3-13, the higher the amount of welan gum added, the higher the overrun obtained, moreover, the ice dispersibility also improved. These improvements are preferable effects because they improve the production efficiency of shaved ice.

Additionally, compared with Comparative Example 3-25, which was formed weak gel to impart a dispersibility to ice, Examples 3-30 and 3-31 exhibited highly improved ice dispersibility even with a low viscosity, and the overrun was also excellent. Example 3-30 exhibited scoopability substantially equivalent to that of Comparative Example 3-25. However, despite its low viscosity, Example 3-30 exhibited an overrun substantially equivalent to that of Comparative Example 3-25. Moreover, although Example 3-31 had an overrun substantially equivalent to that of Comparative Example 3-25, the scoopability was excellent.

Table 3-15

| | |
|---|---|
| Whole-Milk Sweetened Condensed Milk | 24.0 |
| High-Fructose Corn Syrup | 16.0 |
| Sugar | 8.0 |
| Skimmed Milk Powder | 5.5 |
| Emulsifier (HOMOGEN*2 DM-S*1) | 0.05 |
| Stabilizer | See Table 3-16 |
| Flavor (CONDENSED MILK BASE *1) | 0.1 |
| With addition of Ion-Exchanged Water | 100.0% |

Preparation Method

Step 1) While whole-milk sweetened condensed milk, high-fructose corn syrup, and water were stirred, a mixture of sugar, skimmed milk powder, the emulsifier, and a stabilizer prepared beforehand was added thereto, and the mixture was stirred for dissolution at 80°C for 10 minutes.

Step 2) The total amount was adjusted, and homogenized (first step: 10 MPa, second step: 5 MPa), followed by cooling down to 5°C.

Step 3) The flavor was added to the preparation obtained in step 2).

Step 4) Ice was shaved with an ice shaver, and the shaved ice and syrup (the preparation obtained in step 3)) were mixed with a handheld mixer for 30 seconds such that the ratio of ice to syrup was 1/1. The resulting product was packed in a container, and hardened at -40°C.

Table 3-16

| | Stabilizer | Amount (%) | Viscosity (mPa·s) | Overrun (%) | Scoopability |
|---|---|---|---|---|---|
| Blank | - | - | 16 | 12.9 | Hard |
| Example 3-35 | Welan Gum | 0.05 | 67 | 16.5 | Scoopability was better than in the blank and Comparative Example 3-23 |
| Example 3-36 | | 0.1 | 167 | 19.7 | Scoopability was better than in Example 3-35 |
| Example 3-37 | | 0.2 | 410 | 21.8 | Scoopability was slightly better than in Example 3-36 |
| Example 3-38 | | 0.25 | 593 | 24.7 | Scoopability was slightly better than in Example 3-37 |
| Comparative Example 3-26 | Locust Bean Gum Carrageenan | 0.1 0.1 | 205 | 17.6 | Scoopability was better than in the blank |
| Welan Gum: VIS TOP*2 W*1 Locust Bean Gum: VIS TOP*2 D-30*1 Carrageenan: CARRAGEENAN CSI-1(F)*1 | | | | | |

Evaluation

In the case of the formula of milk-flavored shaved ice shown in Table 3-15, as with the formula of soda-flavored shaved ice, the addition of welan gum improved the overrun and scoopability. As the overrun increased, the texture became lighter. The better scoopability made it possible to scoop out shaved ice with less force, making it easier to eat the shaved ice. Moreover, although Example 3-35 exhibited an overrun substantially equivalent to that in Comparative Example 3-26, the scoopability was excellent, and the shaved ice was easier to eat.

Test Example 3-7: Frozen Beverage

In accordance with the formula shown in Table 3-17, frozen beverages were prepared. The melting rate and Brix of the resulting meltwater were measured over time during melting of the prepared frozen beverages. Table 3-18 shows the type and amount of each stabilizer added, and the evaluation results.

Table 3-17

| High-Fructose Corn Syrup | 20.0 |
|---|---|

(continued)

| 5-fold Concentrated Citrus Mixed Fruit Juice | 1.0 |
| Stabilizer | See Table 3-18 |
| Color Additive (Food Blue No.1) | 0.0002 |
| Citric Acid (Anhydrous) | Added until a predetermined pH |
| Flavor (ORANGE EXTRACT No. 51237*1) | 0.1 |
| Color Additive (CAROTENE BASE No. 35468*1) | 0.02 |
| With addition of Ion-Exchanged Water | 100.0% |

Preparation Method

Step 1) While high-fructose corn syrup and ion-exchanged water were stirred, a stabilizer was added thereto, and the mixture was stirred for dissolution at 80°C for 10 minutes.
Step 2) 5-fold concentrated citrus mixed fruit juice, citric acid, the flavor, and the color additive were added to the preparation obtained in step 1), and the total amount was adjusted.
Step 3) The resulting product was heated up to 95°C, and hot water was added to compensate for evaporated water, followed by hot-packing in a 200 ml-PET bottle.
Step 4) After cooling, the product was hardened at -20°C in a thermostatic chamber, thereby obtained a frozen beverage.

Measurement of Change in Brix of Meltwater

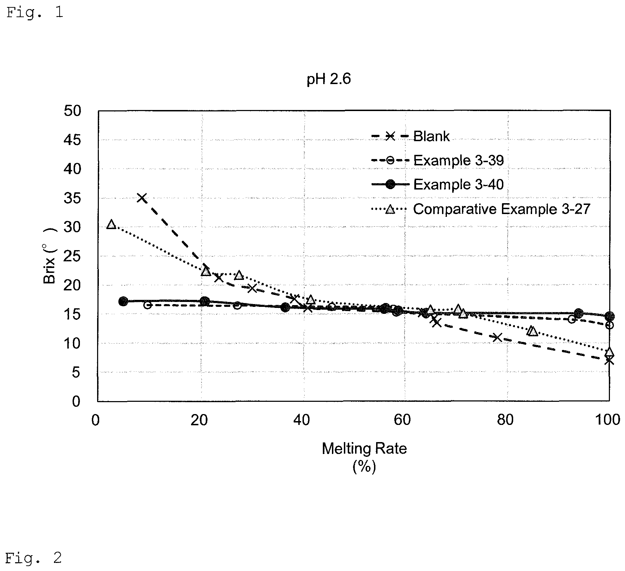

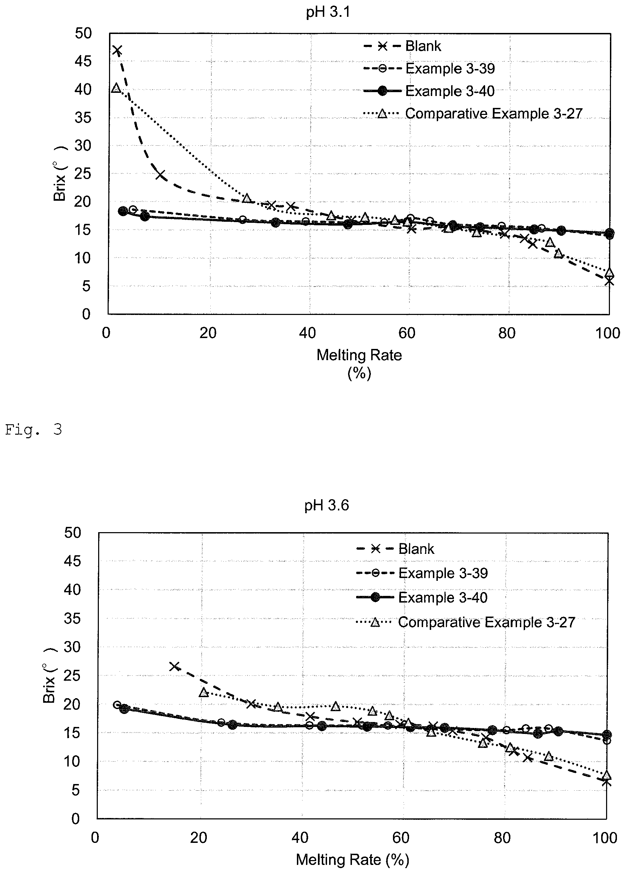

The frozen beverage adjusted to -20°C was opened, and the easiness of squeezing the beverage out of the bottle was evaluated. The evaluation of the easiness of squeezing out the frozen beverage was performed based on the easiness of pushing out the content by squeezing the bottle by hand.
The evaluation is ranked in the order of AA>A>B>B/c>c based on easiness to squeeze out the beverage.
Other PET bottles of the frozen beverages were opened, turned upside-down, and allowed to stand at room temperature (27°C) for 2 hours and 30 minutes. The resulting meltwater of the frozen beverages were collected every 10 minutes, and the Brix was measured with a digital saccharimeter (Atago Co., Ltd., PR-101$\alpha$). The beverages were ranked by measuring the difference in Brix between the beginning and the end of the obtained meltwater in accordance with the following criteria. Table 3-18 shows the results. Figs. 1 to 3 show the Brix values in graphs.

Evaluation Criteria

A: The difference in Brix between the beginning and the end of the obtained meltwater is less than 10.
B: The difference in Brix between the beginning and the end of the obtained meltwater is 10 or more and less than 20.
C: The difference in Brix between the beginning and the end of the obtained meltwater is 20 or more.

Table 3-18

|  | Stabilizer | Amount (%) | pH | Easiness of Squeezing Out the Beverage | Change in Brix | Evaluation |
|---|---|---|---|---|---|---|
| Blank | - | - | 2.6 | C | 28.0 | C |
|  |  |  | 3.1 | C | 41.0 | C |
|  |  |  | 3.6 | C | 20.0 | C |

(continued)

|  | Stabilizer | Amount (%) | pH | Easiness of Squeezing Out the Beverage | Change in Brix | Evaluation |
|---|---|---|---|---|---|---|
| Example 3-39 | Welan Gum (VIS TOP*2 W*1) | 0.05 | 2.6 | B | 3.5 | A |
|  |  |  | 3.1 | B | 4.6 | A |
|  |  |  | 3.6 | B | 6.2 | A |
| Example 3-40 |  | 0.10 | 2.6 | A | 2.7 | A |
|  |  |  | 3.1 | A | 3.8 | A |
|  |  |  | 3.6 | A | 4.5 | A |
| Example 3-41 |  | 0.20 | 2.6 | A | - | A |
| Comparative Example 3-27 | Xanthan Gum (SAN ACE*2 NXG-C*1) | 0.10 | 2.6 | B/C | 22.0 | C |
|  |  |  | 3.1 | B/C | 32.7 | C |
|  |  |  | 3.6 | B/C | 14.4 | B |
| Viscosity Measurement Condition: B-type Rotational Viscometer, 5°C, 60 rpm | | | | | | |

Evaluation Results

In Example 3-39, to which welan gum was added, the frozen beverage was easily squeezed out within the pH range of 2.6 to 3.6 without being affected by the pH change. Additionally, there was substantially no change in the Brix of the meltwater, and the frozen beverage was confirmed to have a consistent taste from the beginning to the end of drinking.

The blank product, however, exhibited a great difference in the Brix between the two points at low melting rate and high melting rate irrespective of pH, and the taste was confirmed to significantly change from the beginning to the end of drinking. The resulting frozen beverage was also difficult to squeeze out from the bottle. Comparative Example 3-27, in which xanthan gum, used in frozen beverages, was added as a stabilizer, exhibited a smaller effect in narrowing the difference in the Brix than the cases where welan gum was used as a stabilizer. The frozen beverage of Comparative Example 3-27 was easier to squeeze out from the bottle than the blank, but was still not in a favorable condition to drink.

Test Example 3-8: Lacto-ice (combinational use of welan gum and a polysaccharide)

In accordance with the formula and preparation method of Test Example 3-1, lacto-ice was prepared. The effects of the use of welan gum and a polysaccharide in combination (shape formability, shape retention, and syneresis suppression) were evaluated.

Evaluation Methods

The obtained lacto-ice was evaluated for shape formability, shape retention, and syneresis suppression.

Evaluation of Shape Formability

The lacto-ice that had been subjected to freezing was packed in a piping bag, and squeezed out into a cone shape on a filter paper. The change in shape over time was observed (after 1 minute and after 5 minutes). Table 3-20 shows the results.

Table 3-19

| Evaluation of Shape Formability | Blank | Example 3-42 | Example 3-43 | Example 3-44 | Example 3-45 | Example 3-46 | Example 3-47 | Example 3-48 | Example 3-49 | Example 3-50 | Example 3-51 |
|---|---|---|---|---|---|---|---|---|---|---|---|
| Welan Gum | - | 0.1 | 0.15 | 0.1 | 0.1 | 0.1 | 0.105 | 0.105 | 0.105 | 0.15 | 0.225 |
| Locust Bean Gum | - | 0.04 | 0.04 | 0.07 | 0.1 | 0.13 | 0.075 | 0.105 | 0.135 | 0.105 | 0.045 |
| Tamarind Seed Gum | - | 0.06 | 0.06 | 0.06 | 0.06 | 0.06 | 0.12 | 0.09 | 0.06 | 0.045 | 0.03 |
| After 1 minute from being squeezed out | 10 | 9 | 10 | 9 | 9 | 9 | 10 | 10 | 10 | 10 | 10 |
| After 5 minutes from being squeezed out | 6 | 8 | 9 | 8 | 8 | 9 | 8 | 8 | 8 | 10 | 10 |
| Appearance Evaluation --- the edge sharpness, surface smoothness, etc., of the squeezed-out lacto-ice were visually observed, and rated on a scale of 1 to 10, with 10 being the best and 1 being the worst (the worst is the state of the blank at the 10-minute point after being squeezed out). <br> Welan Gum: VIS TOP*2 W*1 <br> Locust Bean Gum: VIS TOP*2 D-30*1 <br> Tamarind Seed Gum: VIS TOP*2 D-2032*1 | | | | | | | | | | | |

Evaluation Results

The use of welan gum, locust bean gum, and tamarind seed gum in combination resulted in an appearance substantially equivalent to or slightly inferior to that of the blank at a 1-minute point after being squeezed out, but exhibited a clear difference in shape after 5 minutes, demonstrating that the shape formability is excellent.

Evaluation of Shape Retention

The obtained lacto-ice (stored at -20°C) was taken out and placed on a mesh, and the melting start time (the time point at which the lacto-ice started to drop off) was recorded. Table 3-20 shows the results.

Table 3-20

| Evaluation of Shape Retention | Blank | Example 3-52 | Example 3-53 | Example 3-54 | Example 3-55 | Example 3-56 | Example 3-57 | Example 3-58 | Example 3-59 |
|---|---|---|---|---|---|---|---|---|---|
| Welan Gum | - | 0.1 | 0.02 | 0.1 | 0.1 | 0.105 | 0.105 | 0.105 | 0.045 |
| Locust Bean Gum | - | - | 0.04 | 0.1 | 0.13 | 0.105 | 0.135 | 0.165 | 0.18 |
| Tamarind Seed Gum | - | - | 0.06 | 0.06 | 0.06 | 0.09 | 0.06 | 0.03 | 0.075 |
| Melting Start Time (minutes) | 20 | 30 | 40 | 40 | 50 | 50 | 45 | 40 | 60 |
| Welan Gum: VIS TOP*2 W*1<br>Locust Bean Gum: VIS TOP*2 D-30*1<br>Tamarind Seed Gum: VIS TOP*2 D-2032*1 | | | | | | | | | |

Evaluation Results

The melting start time of the blank product, in which polysaccharides were not added, was 20 minutes. The melting start time of Example 3-52, to which 0.1% of welan gum alone was added, was 30 minutes, indicating that the melting start time was retarded compared with the blank product.

The use of welan gum, locust bean gum, and tamarind seed gum in combination was confirmed to be capable of significantly delaying the melting start time, compared with the blank product and the product in which welan gum alone was added.

Evaluation of Syneresis Suppression

The obtained lacto-ice was placed on a filter paper at room temperature. After 60 minutes, the mass of the filter paper was measured, and the syneresis rate was calculated using the following equation. Table 3-21 shows the results.

```
The syneresis rate = the amount of water absorbed by the filter
paper/the mass of the lacto-ice × 100

(the amount of water absorbed by the filter paper = the mass of
the filter paper after 60 minutes − the initial mass of the
filter paper)
```

Table 3-21

| Evaluation of Syneresis Suppression | Blank | Example 3-60 | Example 3-61 | Example 3-62 | Example 3-63 | Example 3-64 | Example 3-65 | Example 3-66 | Example 3-67 | Example 3-68 | Example 3-69 | Example 3-70 | Example 3-71 |
|---|---|---|---|---|---|---|---|---|---|---|---|---|---|
| Welan Gum | - | 0.1 | 0.15 | 0.1 | 0.1 | 0.1 | 0.105 | 0.105 | 0.105 | 0.105 | 0.045 | 0.15 | 0.225 |
| Locust Bean Gum | - | 0.04 | 0.04 | 0.07 | 0.1 | 0.13 | 0.075 | 0.105 | 0.135 | 0.165 | 0.18 | 0.105 | 0.045 |
| Tamarind Seed Gum | - | 0.06 | 0.06 | 0.06 | 0.06 | 0.06 | 0.12 | 0.09 | 0.06 | 0.03 | 0.075 | 0.045 | 0.03 |
| Syneresis Rate (%) | 17.0 | 3.6 | 3.4 | 3.2 | 2.3 | 2.0 | 2.3 | 1.1 | 1.4 | 1.5 | 2.3 | -2.0 | 2.7 |

Welan Gum: VIS TOP*2 W*1
Locust Bean Gum: VIS TOP*2 D-30*1
Tamarind Seed Gum: VIS TOP*2 D-2032*1

Evaluation Results

The Test Example demonstrated that the use of welan gum, locust bean gum, and tamarind seed gum in combination remarkably suppressed the syneresis, compared with the blank product.

Subsequently, the combinational effect of welan gum and locust bean gum, or welan gum and guar gum were evaluated respectively. In accordance with the formula and preparation method of Test Example 3-1, lacto-ice was prepared. Table 3-22 shows the type and the amount of each polysaccharide added, and the evaluation.

Table 3-22

| Amount (%) | Example 3-72 | Example 3-73 | Example 3-74 | Example 3-75 | Example 3-76 | Example 3-77 | Comparative Example 3-28 | Example 3-77 | Example 3-78 | Example 3-79 | Comparative Example 3-29 |
|---|---|---|---|---|---|---|---|---|---|---|---|
| Welan Gum | 0.2 | 0.16 | 0.12 | 0.1 | 0.08 | 0.04 | - | 0.16 | 0.1 | 0.04 | - |
| Locust Bean Gum | - | 0.04 | 0.08 | 0.1 | 0.12 | 0.16 | 0.2 | - | - | - | - |
| Guar Gum | - | - | - | - | - | - | - | 0.04 | 0.1 | 0.16 | 0.2 |
| Syneresis Rate (%) | 4.3 | 4.6 | 4.6 | 5.3 | 5.1 | 6.6 | 11.5 | 5.4 | 5.5 | 6.9 | 12.9 |
| Shape Formability | AA | AA | A | A | A | B | CC | AA | A | C | CC |
| Viscosity (mPa·s) | 730 | 1193 | 888 | 739 | 791 | 548 | 239 | 680 | 529 | 340 | 188 |
| Flavor and Texture (Sharp aftertaste) | AA | AA | A | A | B | C | CC | AA | A | C | CC |

Evaluation: Excellent AA>A>B>C>CC Poor
Welan Gum: VIS TOP*2 W*1
Locust Bean Gum: VIS TOP*2 D-30*1
Guar Gum: VIS TOP*2 D-20*1

Comprehensive Evaluation

The lacto-ice to which welan gum alone was added (Example 3-72) and the lacto-ice to which welan gum was added in combination with locust bean gum or guar gum (Examples 3-73 to 3-79) exhibited more suppressed syneresis than the lacto-ice to which locust bean gum (Comparative Example 3-28) or guar gum (Comparative Example 3-29) alone was added. The addition of welan gum also resulted in excellent shape formability. The use of welan gum in combination with locust bean gum increased the viscosity, which then enhanced the bodying sensation, indicating that the use of welan gum and locust bean gum in combination imparts a richer texture than the use of welan gum alone or the use of locust bean gum alone.

A similar tendency was observed with the lacto-ice in which welan gum and guar gum were used in combination. The lacto-ice exhibited a less slimy texture, better perceived meltability in the mouth, a cooling sensation, and an excellent bodying sensation, compared with the lacto-ice in which guar gum was used alone. The single use of locust bean gum or guar gum spoils the original flavor of frozen desserts due to the odor derived from the polysaccharide materials; however, the addition of welan gum, which gives a sharp aftertaste, was confirmed to be capable of improving the bodying sensation as well as the flavor, even when added in a small amount in combination.

## Claims

1. A foam-containing composition comprising welan gum.

2. A method for stabilizing foam, the method comprising adding welan gum to a foam-containing composition.

3. A stabilizer for a foam-containing composition, the stabilizer comprising welan gum.

4. An egg white-containing baked confection comprising welan gum.

5. A method for improving a texture of an egg white-containing baked confection, the method comprising adding welan gum.

6. A method for producing an egg white-containing baked confection, the method comprising adding welan gum.

7. A texture-improving agent for an egg white-containing baked confection, the texture-improving agent comprising welan gum.

8. A frozen dessert comprising welan gum.

9. A method for stabilizing a frozen dessert, the method comprising adding welan gum.

10. A method for producing a frozen dessert, the method comprising adding welan gum.

Fig. 1

pH 2.6

Fig. 2

## pH 3.1

Fig. 3

## pH 3.6

## INTERNATIONAL SEARCH REPORT

| International application No. |
| --- |
| PCT/JP2016/075915 |

**A. CLASSIFICATION OF SUBJECT MATTER**
*A23L29/20*(2016.01)i, *A23G9/04*(2006.01)i, *A23G9/32*(2006.01)i, *A23G9/44* (2006.01)i, *A23G9/52*(2006.01)i, *A23L15/00*(2016.01)i

According to International Patent Classification (IPC) or to both national classification and IPC

**B. FIELDS SEARCHED**

Minimum documentation searched (classification system followed by classification symbols)
A23L29/20, A23G9/04, A23G9/32, A23G9/44, A23G9/52, A23L15/00

Documentation searched other than minimum documentation to the extent that such documents are included in the fields searched
Jitsuyo Shinan Koho          1922-1996   Jitsuyo Shinan Toroku Koho   1996-2016
Kokai Jitsuyo Shinan Koho    1971-2016   Toroku Jitsuyo Shinan Koho   1994-2016

Electronic data base consulted during the international search (name of data base and, where practicable, search terms used)
JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2009-112219 A (Asahi Kasei Chemicals Corp.), 28 May 2009 (28.05.2009), paragraphs [0001], [0020], [0032] to [0033] (Family: none) | 1-10 |
| X/A | JP 7-147905 A (San-Ei Gen F.F.I., Inc.), 13 June 1995 (13.06.1995), paragraphs [0006] to [0010] (Family: none) | 1-7/8-10 |
| X/A | JP 9-205995 A (Kao Corp.), 12 August 1997 (12.08.1997), paragraphs [0003], [0007], [0016] (Family: none) | 1-3,8-10/4-7 |

☒ Further documents are listed in the continuation of Box C.          ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 26 September 2016 (26.09.16) | 04 October 2016 (04.10.16) |

| Name and mailing address of the ISA/ | Authorized officer |
| --- | --- |
| Japan Patent Office 3-4-3,Kasumigaseki,Chiyoda-ku, Tokyo 100-8915,Japan | Telephone No. |

Form PCT/ISA/210 (second sheet) (January 2015)

**INTERNATIONAL SEARCH REPORT**

International application No.

PCT/JP2016/075915

C (Continuation).   DOCUMENTS CONSIDERED TO BE RELEVANT

| Category* | Citation of document, with indication, where appropriate, of the relevant passages | Relevant to claim No. |
|---|---|---|
| X/A | JP 2011-147357 A   (Shin-Etsu Chemical Co., Ltd.), 04 August 2011 (04.08.2011), paragraphs [0001], [0013] to [0014] (Family: none) | 1-3/4-10 |
| X/A | JP 2009-159908 A   (Asahi Kasei Chemicals Corp.), 23 July 2009 (23.07.2009), paragraphs [0001], [0024], [0026], [0031] (Family: none) | 8-10/1-7 |
| P,X | Kazuhiro MAEDA et al., "Shokuhin Kaihatsu no Kanosei o Hirogeru Shinki Tatorui 'Welangum'", FFI Journal, 01 May 2016 (01.05.2016), vol.221, no.2, page 179 | 1-10 |
| P,X | Kazuhiro MAEDA, FFI Reports "Welangum no Shokuhin eno Oyo", FFI Journal, 01 August 2016 (01.08.2016), vol.221, no.3, pages 254 to 259 | 1-10 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3806605 A
- EP 354356 A
- JP H07236443 A
- JP H07108201 B
- JP 2005295841 A
- JP H04356160 A
- JP H10295339 A
- JP H1156244 A
- JP 2000060507 A
- JP 2000157232 A
- JP S6167467 A
- JP 2004016148 A
- JP 2010508038 A
- JP H07252494 A
- JP 2011057747 A

- JP 2003073229 A
- JP H09143498 A
- JP 2011225763 A
- JP H11148041 A
- JP S63315547 A
- JP H0655529 A
- JP 2009249533 A
- JP 2009298809 A
- JP S62190050 A
- JP S4410149 B
- JP S52114054 A
- JP S5737306 B
- JP 2002360178 A
- JP 2002253126 A

**Non-patent literature cited in the description**

- **NAOMICHI OKAZAKI ; MASAO SANO.** Shokuhin-tatorui Nyuka, Zonen, Geruka No Chishiki (Food Polysaccharides, Knowledge of Emulsification, Thickening, and Gel Formation). Saiwai Shobo, 2001